# EUROPEAN PATENT SPECIFICATION

(11) **EP 3 691 638 B1**
(45) Date of publication and mention of the grant of the patent: **11.12.2024**
(21) Application number: 18797221.1
(22) Date of filing: 05.10.2018
(51) Int. Cl.: A61K 31/4245, A61K 31/47, A61K 31/4709, A61P 11/00

(54) **COMPOUNDS, COMPOSITIONS AND METHODS FOR INCREASING CFTR ACTIVITY**
VERBINDUNGEN, ZUSAMMENSETZUNGEN UND VERFAHREN ZUR STEIGERUNG DER CFTR-AKTIVITÄT
COMPOSÉS, COMPOSITIONS ET MÉTHODES POUR AUGMENTER L'ACTIVITÉ DE CFTR

(30) Priority: 06.10.2017 US 201762569204 P
(43) Date of publication of application: 12.08.2020
(73) Proprietor: Proteostasis Therapeutics, Inc., Boston, MA 02135 (US)
(72) Inventor: PARKS, Daniel, Pepperell, MA 01463 (US); MUNOZ, Benito, Newtonville, MA 02460 (US); BASTOS, Cecilia, M., South Grafton, MA 01560 (US)
(74) Representative: Harris, Jennifer Lucy
(86) International application number: PCT/US2018/054526
(87) International publication number: WO 2019/071078

(56) References cited:
- WO-A1-2017/019589
- WO-A1-2017/062581
- WO-A1-2017/151725
- WO-A1-2017/177124
- US-A1- 2006 074 075
- SHOWELL G A ET AL: "CHEMISTRY CHALLENGES IN LEAD OPTIMIZATION: SILICON ISOSTERES IN DRUG DISCOVERY", DRUG DISCOVERY TODAY, ELSEVIER, AMSTERDAM, NL, vol. 8, no. 12, 15 June 2003 (2003-06-15), pages 551 - 556, XP001188984, ISSN: 1359-6446, DOI: 10.1016/S1359-6446(03)02726-0

## Description

### BACKGROUND

Cells normally maintain a balance between protein synthesis, folding, trafficking, aggregation, and degradation, referred to as protein homeostasis, utilizing sensors and networks of pathways (Sitia et al., Nature 426: 891-894, 2003; Ron et al., Nat Rev Mol Cell Biol 8: 519-529, 2007). The cellular maintenance of protein homeostasis, or proteostasis, refers to controlling the conformation, binding interactions, location and concentration of individual proteins making up the proteome. Protein folding *in vivo* is accomplished through interactions between the folding polypeptide chain and macromolecular cellular components, including multiple classes of chaperones and folding enzymes, which minimize aggregation (Wiseman et al., Cell 131: 809-821, 2007). Whether a given protein folds in a certain cell type depends on the distribution, concentration, and subcellular localization of chaperones, folding enzymes, metabolites and the like. Cystic fibrosis and other maladies of protein misfolding arise as a result of an imbalance in the capacity of the protein homeostasis (proteostasis) environment to handle the reduced energetic stability of misfolded, mutated proteins that are critical for normal physiology (Balch et al., Science 319, 916-9 (2008); Powers, et al., Annu Rev Biochem 78, 959-91 (2009); Hutt et al., FEBS Lett 583, 2639-46 (2009)).

Cystic Fibrosis (CF) is caused by mutations in the cystic fibrosis transmembrane conductance regulator (CFTR) gene which encodes a multi-membrane spanning epithelial chloride channel (Riordan et al., Annu Rev Biochem 77, 701-26 (2008)). Approximately ninety percent of patients have a deletion of phenylalanine (Phe) 508 (ΔF508) on at least one allele. This mutation results in disruption of the energetics of the protein fold leading to degradation of CFTR in the endoplasmic reticulum (ER). The ΔF508 mutation is thus associated with defective folding and trafficking, as well as enhanced degradation of the mutant CFTR protein (Qu et al., JBiol Chem 272, 15739-44 (1997)). The loss of a functional CFTR channel at the plasma membrane disrupts ionic homeostasis (Cl⁻, Na⁺, HCO₃⁻) and airway surface hydration leading to reduced lung function (Riordan et al.). Reduced periciliary liquid volume and increased mucus viscosity impede mucociliary clearance resulting in chronic infection and inflammation, phenotypic hallmarks of CF disease (Boucher, J Intern Med 261, 5-16 (2007)). In addition to respiratory dysfunction, ΔF508 CFTR also impacts the normal function of additional organs (pancreas, intestine, gall bladder), suggesting that the loss-of-function impacts multiple downstream pathways that will require correction.

In addition to cystic fibrosis, mutations in the CFTR gene and/or the activity of the CFTR channel has also been implicated in other conditions, including for example, congenital bilateral absence of vas deferens (CBAVD), acute, recurrent, or chronic pancreatitis, disseminated bronchiectasis, asthma, allergic pulmonary aspergillosis, smoking-related lung diseases, such as chronic obstructive pulmonary disease (COPD), dry eye disease, Sjogren's syndrome and chronic sinusitis, cholestatic liver disease (e.g. Primary biliary cirrhosis (PBC) and primary sclerosing cholangitis (PSC)) (Sloane et al. (2012), PLoS ONE 7(6): e39809.doi:10.1371/joumal. pone.0039809; Bombieri et al. (2011), J Cyst Fibros. 2011 Jun; 10 Suppl 2:S86-102; (Albert et al. (2008), Clinical Respiratory Medicine, Third Ed., Mosby Inc.; Levin et al. (2005), Invest Ophthalmol Vis Sci., 46(4):1428-34; Froussard (2007), Pancreas 35(1): 94-5), Son et al. (2017) J Med Chem 60(6):2401-10.

There remains a need in the art for compounds, compositions and methods of increasing CFTR activity as well as for methods of treating CF, other CFTR-related diseases, and other maladies of protein misfolding.

WO 2017/019589 A1 relates to compounds which can increase cystic fibrosis transmembrane conductance regulator (CFTR) activity as measured in human bronchial epithelial (hBE) cells.

WO 2017/062581 A1 relates to compounds that modulate, e.g., address underlying defects in cellular processing of CFTR activity.

US 2006/074075 A1 relates to modulators of ATP-Binding Cassette ("ABC") transporters or fragments thereof, including Cystic Fibrosis Transmembrane Conductance Regulator, compositions thereof, and methods therewith.

WO 2017/151725 A1 relates to direct blend formulations comprising 3-chloro-4-(6- hydroxyquinolin-2-yl)benzoic acid and a method of treating or lessening the severity of cystic fibrosis in a patient, comprising the step of stabilizing the CFTR protein both in the cell and at the cell membrane by administering to said patient an effective amount of an inhibitor of S-nitrosoglutathione reductase (GSNOR).

Showell G. et al, Drug Discovery Today, 2003, Vol. 8(12), pp. 551-556 relates generally to the introduction of biosteres, such as by sila-substitution, into lead molecules to improve their properties in the drug design process.

WO 2017/177124 A1 relates to compounds that modulate e.g., address underlying defects in cellular processing of CFTR activity.

### SUMMARY

The present disclosure is based, in part, on the discovery that disclosed compounds such as those having the Formulae Ia, Ib, Ic, Id, II, and III increase cystic fibrosis transmembrane conductance regulator (CFTR) activity as measured in human bronchial epithelial (hBE) cells.

The present invention, which is defined by the appended claims, relates to aspects and embodiments as described herein wherein a compound of formula Ia, Ib, Ic or Id is: 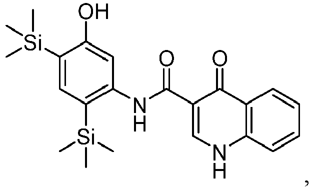
or a pharmaceutically acceptable salt thereof;
a compound of formula II is: 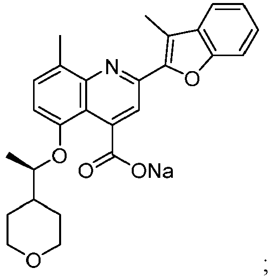
and a compound of formula III or IV is: 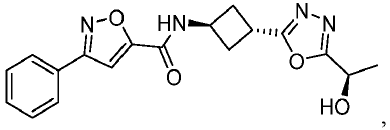
or a pharmaceutically acceptable salt thereof.

In one aspect, the invention provides a compound represented by 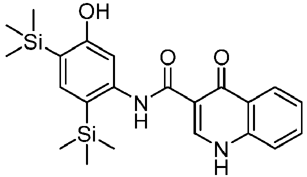 or a pharmaceutically acceptable salt thereof, for use in a method of treating a disease associated with decreased cystic fibrosis transmembrane conductance regulator (CFTR) activity in a subject in need thereof, the method comprising administering said compound in combination with compounds: 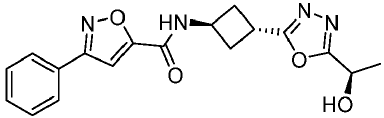 or a pharmaceutically acceptable salt thereof, and 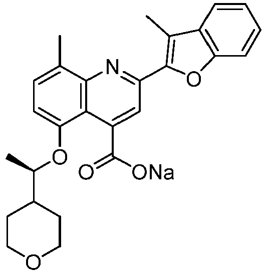 wherein the disease is selected from the group consisting of cystic fibrosis, congenital bilateral absence of vas deferens (CBAVD), acute, recurrent, or chronic pancreatitis, disseminated bronchiectasis, asthma, allergic pulmonary aspergillosis, chronic obstructive pulmonary disease (COPD), chronic sinusitis, dry eye disease, protein C deficiency, A-β-lipoproteinemia, lysosomal storage disease, type 1 chylomicronemia, mild pulmonary disease, lipid processing deficiencies, type 1 hereditary angioedema, coagulation-fibrinolyis, hereditary hemochromatosis, CFTR-related metabolic syndrome, chronic bronchitis, constipation, pancreatic insufficiency, hereditary emphysema, Sjogren's syndrome, familial hypercholesterolemia, I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, nephrogenic DI, Charcot-Marie Tooth syndrome, Perlizaeus-Merzbacher disease, Alzheimer's disease, Parkinson's disease, amyotrophic lateral sclerosis, progressive supranuclear palsy, Pick's disease, Huntington's disease, spinocerebellar ataxia type I, spinal and bulbar muscular atrophy, dentatorubral pallidoluysian, myotonic dystrophy, hereditary Creutzfeldt-Jakob disease (due to prion protein processing defect), Fabry disease, cholestatic liver disease (primary biliary cirrhosis (PBC), primary sclerosing cholangitis (PSC)), and Straussler-Scheinker syndrome.

In another aspect, the invention provides a CFTR amplifier compound for use in a method of treating cystic fibrosis in a patient homozygous for the ΔF508 mutation or having a ΔF508/G542X mutation, the method comprising administering to the patient an effective combination, sequentially or substantially simultaneously, of the CFTR amplifier compound, a CFTR corrector compound, and a CFTR potentiator compound, wherein:
the amplifier compound is represented by: 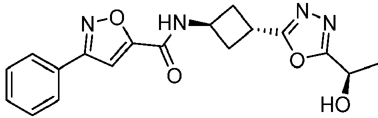 or a pharmaceutically acceptable salt thereof;
the corrector compound is represented by: 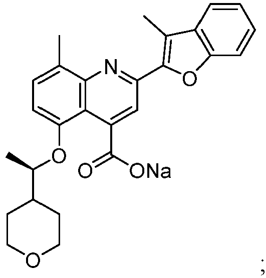 and
the potentiator compound is represented by: 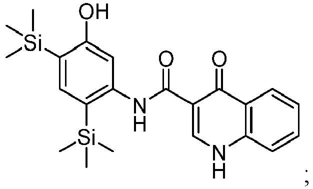
or a pharmaceutically acceptable salt thereof. The technical information set out below may in some respects go beyond the scope of the present invention, which is defined by the appended claims. The additional technical information is provided to place the present invention in a broader technical context and to illustrate possible related technical developments.

The references to the methods of treatment by therapy or surgery or in vivo diagnostic methods herein are to be interpreted as references to compounds, pharmaceutical compositions, combinations and/or medicaments for use in those methods.

In an embodiment, this disclosure is at least partially directed to a method of enhancing cystic fibrosis transmembrane conductance regulator (CFTR) activity in a subject in need thereof, which includes administering to said subject a therapeutically effective amount of a first compound of Formula Ia, Ib, Ic or Id, a second compound of Formula II, and a third compound of Formula III or IV, as disclosed herein.

In additional embodiments, a method of enhancing (e.g., increasing) cystic fibrosis transmembrane conductance regulator (CFTR) activity in a subject in need thereof is provided, comprising administering to said subject a therapeutically effective amount of a first compound of Formula Ia, Ib, Ic or Id, a second compound of Formula II, and a third compound of Formula III or IV, as disclosed herein.

In certain of these embodiments, the activity of one or more (e.g., one or two) mutant CFTRs (e.g., ΔF508, S549N, G542X, G551D, R117H, N1303K, W1282X, R553X, 621+1G>T, 1717-1G>A, 3849+10kbC>T, 2789+5G>A, 3120+1G>A, I507del, R1162X, 1898+1G>A, 3659delC, G85E, D1152H, R560T, R347P, 2184insA, A455E, R334W, Q493X, E56K, P67L, R74W, D110E, D110H, R117C, G178R, E193K, L206W, R347H, R352Q, A455E, S549R, G551S, D579G, S945L, S997F, F1052V, K1060T, A1067T, G1069R, R1070Q, R1070W, F1074L, G1244E, S1251N, S1255P, D1270N, G1349D, and 2184delA CFTR) is enhanced (e.g., increased). In certain embodiments, ΔF508 CFTR activity is enhanced (e.g., increased). In other embodiments, the activities of two mutant CFTRs (e.g., ΔF508 and G551D; ΔF508 and A455E; or G542X; Δ508F) are enhanced (e.g., increased).

In certain embodiments, a method is provided comprising administering a first compound of Formula Ia, Ib, Ic or Id, a second compound of Formula II, and a third compound of Formula III or IV, as disclosed herein, to a subject (e.g., a human patient) suffering from a disease associated with decreased CFTR activity (e.g., cystic fibrosis, congenital bilateral absence of vas deferens (CBAVD), acute, recurrent, or chronic pancreatitis, disseminated bronchiectasis, asthma, allergic pulmonary aspergillosis, chronic obstructive pulmonary disease (COPD), chronic sinusitis, dry eye disease, protein C deficiency, A-β-lipoproteinemia, lysosomal storage disease, type 1 chylomicronemia, mild pulmonary disease, lipid processing deficiencies, type 1 hereditary angioedema, coagulation-fibrinolyis, hereditary hemochromatosis, CFTR-related metabolic syndrome, chronic bronchitis, constipation, pancreatic insufficiency, hereditary emphysema, Sjogren's syndrome, familial hypercholesterolemia, I-cell disease/pseudo-Hurler, mucopolysaccharidoses, Sandhof/Tay-Sachs, Crigler-Najjar type II, polyendocrinopathy/ hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, nephrogenic DI, Charcot-Marie Tooth syndrome, Perlizaeus-Merzbacher disease, Alzheimer's disease, Parkinson's disease, amyotrophic lateral sclerosis, progressive supranuclear palsy, Pick's disease, Huntington's disease, spinocerebellar ataxia type I, spinal and bulbar muscular atrophy, dentatorubral pallidoluysian, myotonic dystrophy, hereditary Creutzfeldt-Jakob disease (due to prion protein processing defect), Fabry disease, cholestatic liver disease (e.g. Primary biliary cirrhosis (PBC) and primary sclerosing cholangitis (PSC)), and Straussler-Scheinker syndrome). In certain embodiments, the disease is cystic fibrosis.

In yet additional embodiments, this disclosure is directed to treating a patient suffering from cystic fibrosis comprising administering to said patient an effective amount of a first compound of Formula Ia, Ib, Ic or Id,, a second compound of Formula II, and a third compound of Formula III or IV, as disclosed herein.

In another embodiment, this disclosure provides methods of treating cystic fibrosis in a patient homozygous for the ΔF508 mutation or having a ΔF508/G542X mutation, comprising administering to the patient an effective combination, sequentially or substantially simultaneously, of a CFTR amplifier compound, a CFTR corrector compound, and a CFTR potentiator compound, as disclosed herein.

In some embodiments, the methods described herein can further include administering an additional CFTR modulator. In certain embodiments, the additional CFTR modulator is a CFTR corrector (e.g., VX-809 (lumacaftor), deuterated lumacaftor, VX-661 (tezacaftor), deuterated tezacaftor, VX-983, VX-152, VX-440, VX-445, VX-659, GLPG2851, GLPG2665, GLPG2737, GLPG3221, or GLPG2222).

### BRIEF DESCRIPTION OF THE DRAWINGS

FIG. 1 depicts the *in vitro* CFTR modulating effects of doublet and triplet combinations of disclosed CFTR modulators in CFTR homozygous F508del patient cells.
FIG. 2 depicts the *in vitro* CFTR modulating effects of doublet and triplet combinations of disclosed CFTR modulators in CFTR heterozygous F508del/G542X patient cells.

### DETAILED DESCRIPTION

As used herein, the words "a" and "an" are meant to include one or more unless otherwise specified. For example, the term "an agent" encompasses both a single agent and a combination of two or more agents.

As discussed above, the present disclosure is directed in part to methods of treating CFTR that include administering a first compound of Formula Ia, Ib, Ic or Id, a second compound of Formula II, and a third compound of Formula III or IV, as disclosed herein, or a pharmaceutically acceptable salt, prodrug or solvate thereof.

Disclosed herein are methods of enhancing cystic fibrosis transmembrane conductance regulator (CFTR) activity in a subject in need thereof, comprising administering to said subject a therapeutically effective amount of:
a) a first compound represented by formula Ia, Ib, Ic or Id: 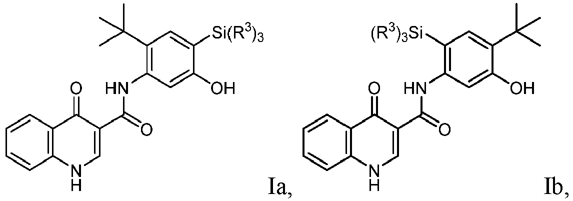 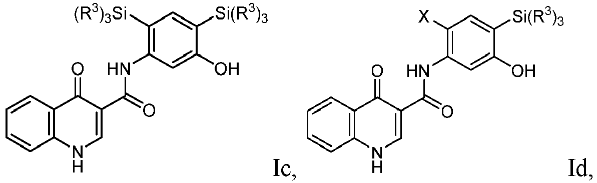 or a pharmaceutically acceptable salt thereof, wherein:
   R³ is independently selected for each occurrence from the group consisting of hydroxyl, C₁₋₄alkyl, C₁₋₄alkoxy, and phenyl, wherein C₁₋₄alkyl, C₁₋₄alkoxy, and phenyl may optionally be substituted by one, two, three or more deuterium atoms; or two R³ groups together with the silicon to which they are attached form a 4-6 membered saturated cyclosilane; and
   X is CF₃ or halogen;
b) a second compound represented by formula II: 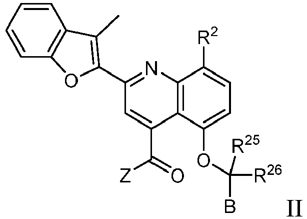 or a pharmaceutically acceptable salt thereof, wherein:
   R² is selected from the group consisting of hydrogen, halogen, cyano, C₁₋₆alkyl, C₁₋₆alkoxy, and C₃₋₆cycloalkyl;
   R²⁵ and R²⁶ are each independently selected from the group consisting of hydrogen and C₁₋₆alkyl;
   B is a 4-10 membered monocyclic, bridged bicyclic, or spirocyclic heterocyclic ring having one or two heteroatoms each independently selected from the group consisting of O, N, and S; wherein if said heterocyclic ring contains an -NH moiety, that nitrogen may optionally be substituted by a substituent selected from the group consisting of C₁₋₆alkyl,-C(O)-C₁₋₆alkyl, -C(O)-O-C₁₋₆alkyl, and -S(O)_{w}-C₁₋₃alkyl (where w is 0, 1, or 2); and wherein said heterocyclic ring may optionally be substituted by one, two, three, or four substituents each independently selected from hydroxyl, C₁₋₆alkyl, C₁₋₆alkoxy, and oxo;
   Z is selected from the group consisting of -OH and -NHR^{Z}; and
   R^{Z} is selected from the group consisting of C₁₋₆alkyl, C₃₋₆cycloalkyl, morpholinyl, pyrrolidinyl, phenyl, pyridinyl, pyrrazolyl and thiazolyl wherein C₁₋₆alkyl, C₃₋₆cycloalkyl, morpholinyl, pyrrolidinyl, phenyl, pyridinyl, pyrrazolyl and thiazolyl may optionally be substituted by one, two, or three substituents each independently selected from the group consisting of halogen, hydroxyl, and -NH₂; and
c) a third compound represented by formula III or formula IV: 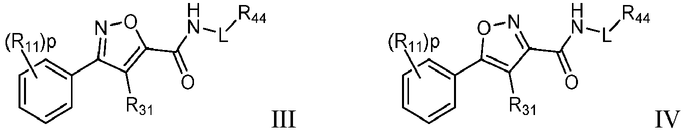
   or a pharmaceutically acceptable salt thereof, wherein:
      p is 1, 2, or 3;
      R₁₁ is independently selected for each occurrence from the group consisting of hydrogen, halogen, and C₁₋₄ alkyl (optionally substituted by one, two or three halogens);
      R₃₁ is selected from the group consisting of hydrogen, halogen, and C₁₋₄alkyl;
      L is selected from the group consisting of C₁₋₆ alkylene, C₃₋₆ cycloalkylene, and C₃₋₆ cycloalkylene-C₁₋₄ alkylene;
      R₄₄ is selected from the group consisting of: 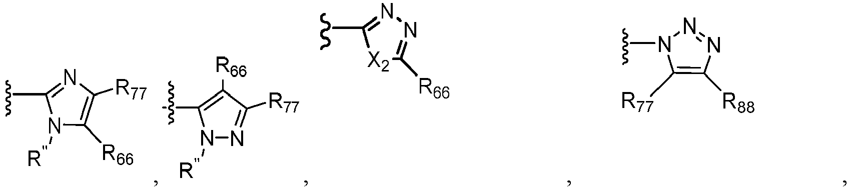 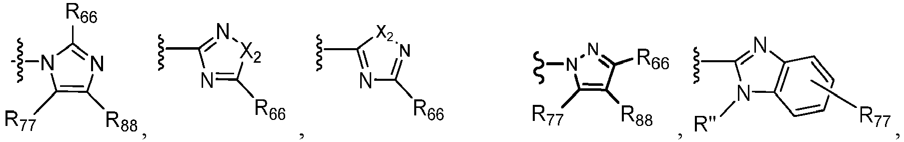
   wherein
      X₂ is O, S, or NRₕₕ;
      R" and R' are each independently selected from H or C₁₋₄alkyl;
      each R₆₆, R₇₇, R₈₈ and R₉₉ is independently selected for each occurrence from Hand R_{gg};
      R_{gg} is selected for each occurrence from the group consisting of halogen, hydroxyl, cyano, -NR'R", C₁₋₆ alkyl, C₃₋₆ cycloalkyl, and C₁₋₆ alkenyl (wherein C₁₋₆ alkyl, C₃₋₆ cycloalkyl, and C₁₋₆ alkenyl are each optionally substituted by one, two, or three substituents each independently selected from halogen, hydroxyl, and C₁₋₆ alkoxy);
      Rₕₕ is selected for each occurrence from the group consisting of H and C₁₋₆ alkyl; and
      R' and R" are each independently selected for each occurrence from Hand C₁₋₄ alkyl.

In certain embodiments, the patient has one or more CFTR mutations selected from the group consisting of ΔF508, S549N, G542X, G551D, R117H, N1303K, W1282X, R553X, 621+1G>T, 1717-1G>A, 3849+10kbC>T, 2789+5G>A, 3120+1G>A, I507del, R1162X, 1898+1G>A, 3659delC, G85E, D1152H, R560T, R347P, 2184insA, A455E, R334W, Q493X, E56K, P67L, R74W, D110E, D110H, R117C, G178R, E193K, L206W, R347H, R352Q, A455E, S549R, G551S, D579G, S945L, S997F, F1052V, K1060T, A1067T, G1069R, R1070Q, R1070W, F1074L, G1244E, S1251N, S1255P, D1270N, G1349D, and 2184delA CFTR.

In certain embodiments, the patient has a ΔF508 and a G542X mutation. In other embodiments, the patient has a homozygous ΔF508 mutation.

In some embodiments, the subject is suffering from a disease associated with decreased CFTR activity. For example, the disease is selected from the group consisting of, e.g., cystic fibrosis, congenital bilateral absence of vas deferens (CBAVD), acute, recurrent, or chronic pancreatitis, disseminated bronchiectasis, asthma, allergic pulmonary aspergillosis, chronic obstructive pulmonary disease (COPD), chronic sinusitis, dry eye disease, protein C deficiency, A-β-lipoproteinemia, lysosomal storage disease, type 1 chylomicronemia, mild pulmonary disease, lipid processing deficiencies, type 1 hereditary angioedema, coagulation-fibrinolyis, hereditary hemochromatosis, CFTR-related metabolic syndrome, chronic bronchitis, constipation, pancreatic insufficiency, hereditary emphysema, Sjogren's syndrome, familial hypercholesterolemia, I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, nephrogenic DI, Charcot-Marie Tooth syndrome, Perlizaeus-Merzbacher disease, Alzheimer's disease, Parkinson's disease, amyotrophic lateral sclerosis, progressive supranuclear palsy, Pick's disease, Huntington's disease, spinocerebellar ataxia type I, spinal and bulbar muscular atrophy, dentatorubral pallidoluysian, myotonic dystrophy, hereditary Creutzfeldt-Jakob disease (due to prion protein processing defect), Fabry disease, cholestatic liver disease, and Straussler-Scheinker syndrome. For example, the disease is cystic fibrosis.

In certain embodiments, the subject is a human patient.

In certain embodiments, a method disclosed herein comprises administering an effective amount of the following compounds to the patient: 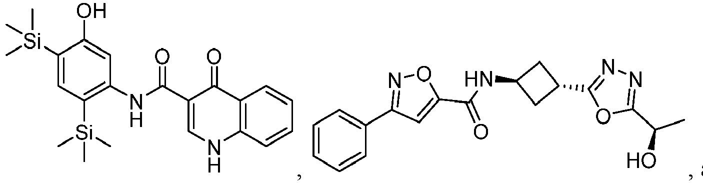 and

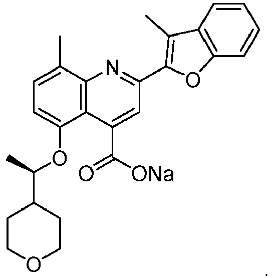

For example, disclosed herein is a method of enhancing cystic fibrosis transmembrane conductance regulator (CFTR) activity in a subject in need thereof, comprising administering to said subject a therapeutically effective amount of the following compounds to the patient: 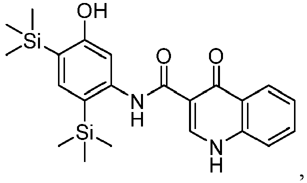 and a compound selected from the group consisting of:

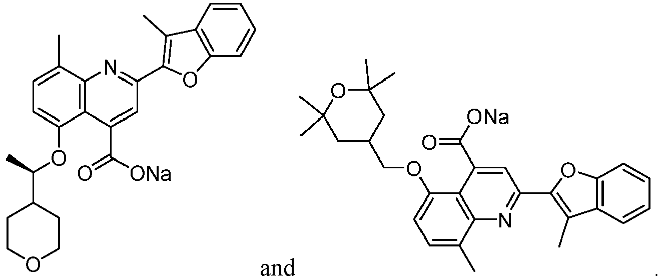

Also provided herein are methods of treating cystic fibrosis in a patient homozygous for the ΔF508 mutation or having a ΔF508/G542X mutation, comprising administering to the patient an effective combination, sequentially or substantially simultaneously, of a CFTR amplifier compound, a CFTR corrector compound and CFTR potentiator compound, wherein:
the amplifier compound is represented by: 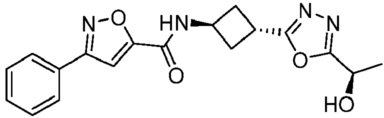 or a pharmaceutically acceptable salt thereof;
the corrector compound is represented by: 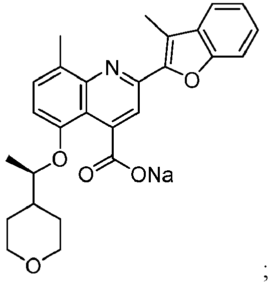 and
the potentiator compound is represented by: 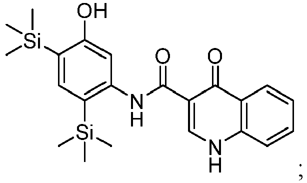
or a pharmaceutically acceptable salt thereof.

Further disclosed herein are methods of treating cystic fibrosis in a patient homozygous for the ΔF508 mutation or having a ΔF508/G542X mutation, comprising administering to the patient an effective combination, sequentially or substantially simultaneously, of a CFTR corrector compound and a CFTR potentiator compound, wherein:
the corrector compound is represented by: 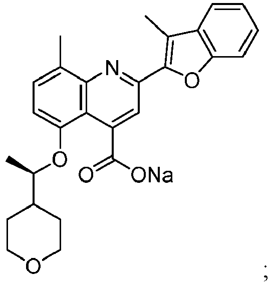 and
the potentiator compound is represented by: 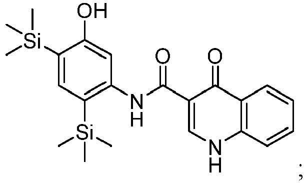
or a pharmaceutically acceptable salt thereof.

Further disclosed herein are methods of treating cystic fibrosis in a patient homozygous for the ΔF508 mutation or having a ΔF508/G542X mutation, comprising administering to the patient an effective combination, sequentially or substantially simultaneously, of a CFTR amplifer compound and a CFTR potentiator compound, wherein:
the amplifier compound is represented by: 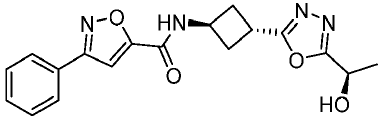 or a pharmaceutically acceptable salt thereof; and
the potentiator compound is represented by: 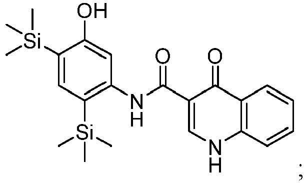
or a pharmaceutically acceptable salt thereof.

In certain embodiments, a method disclosed herein further comprises administering an additional CFTR modulator, as described anywhere herein. In some embodiments, the CFTR modulator is a CFTR corrector. For example, the CFTR corrector may be selected from the group consisting of (3-(6-(1-(2,2-difluorobenzo[d][1,3]dioxol-S-yl)cyclopropanecarboxamido)-3-methylpyridin-2-yl)benzoic acid (lumacaftor), deuterated lumacaftor, ((R)-1-(2,2-difluorobenzo[d][1,3]dioxol-5-yl)-N-(1-(2,3 -dihydroxypropyl)-6-fluoro-2-(1-hydroxy-2-methylpropan-2-yl)-1H-indol-5-yl)cyclopropane-1-carboxamide (tezacaftor), deuterated tezacaftor, VX-152, VX-440, VX-445, VX-659, GLPG2222, GLPG2851, GLPG2737, GLPG3221 and VX-983.

It is to be understood that the specific embodiments described herein can be taken in combination with other specific embodiments delineated herein.

The features and other details of the disclosure will now be more particularly described. Before further description of the present disclosure, certain terms employed in the specification, examples and appended claims are collected here. These definitions should be read in light of the remainder of the disclosure and as understood by a person of skill in the art. Unless defined otherwise, all technical and scientific terms used herein have the same meaning as commonly understood by a person of ordinary skill in the art.

It will be appreciated that the description of the present disclosure herein should be construed in congruity with the laws and principals of chemical bonding.

The term "alkyl", as used herein, unless otherwise indicated, refers to both branched and straight-chain saturated aliphatic hydrocarbon groups having the specified number of carbon atoms; for example, "C₁-C₁₀ alkyl" denotes alkyl having 1 to 10 carbon atoms, and straight or branched hydrocarbons of 1-6, 1-4, or 1-3 carbon atoms, referred to herein as C₁₋₆ alkyl, C₁₋₄ alkyl, and C₁₋₃ alkyl, respectively. Examples of alkyl include, but are not limited to, methyl, ethyl, n-propyl, i-propyl, n-butyl, i-butyl, sec-butyl, t-butyl, n-pentyl, n-hexyl, 2-methylbutyl, 2-methylpentyl, 2-ethylbutyl, 3-methylpentyl, and 4-methylpentyl.

The term, "alkenyl", as used herein, refers to both straight and branched-chain moieties having the specified number of carbon atoms and having at least one carbon-carbon double bond. Exemplary alkenyl groups include, but are not limited to, a straight or branched group of 2-6 or 3-4 carbon atoms, referred to herein as C₂₋₆ alkenyl, and C₃₋₄ alkenyl, respectively. Exemplary alkenyl groups include, but are not limited to, vinyl, allyl, butenyl, pentenyl, etc.

The term, "alkynyl", as used herein, refers to both straight and branched-chain moieties having the specified number or carbon atoms and having at least one carbon-carbon triple bond.

The term "cycloalkyl," as used herein, refers to saturated alkyl cyclic structures, including monocyclic, bridged bicyclic, fused bicyclic, or spirocyclic structures having 3 or more carbon atoms, for example, 3-10, 3-6, or 4-6 carbons, referred to herein as C₃₋₁₀ cycloalkyl, C₃₋₆ cycloalkyl or C₄₋₆ cycloalkyl, whose rings mayrespectively for example, examples of cycloalkyl include, but are not limited to, cyclopropyl, cyclobutyl, cyclopentyl, cyclohexyl, cycloheptyl and adamantyl.

The term "cycloalkenyl," as used herein, refers to cyclic alkenyl moieties having 3 or more carbon atoms.

The term "cycloalkynyl," as used herein, refers to cyclic alkynyl moieties having 5 or more carbon atoms.

"Alkylene" means a straight or branched, saturated aliphatic divalent radical having the number of carbons indicated. "Cycloalkylene" refers to a divalent radical of carbocyclic saturated hydrocarbon group having the number of carbons indicated.

The term "alkoxy" as used herein refers to a straight or branched alkyl group attached to oxygen (alkyl-O-). Exemplary alkoxy groups include, but are not limited to, alkoxy groups of 1-6 or 2-6 carbon atoms, referred to herein as C₁₋₆ alkoxy, and C₂₋₆ alkoxy, respectively. Exemplary alkoxy groups include, but are not limited to methoxy, ethoxy, isopropoxy, etc.

The term "heterocyclic" or "heterocycle" encompasses heterocycloalkyl, heterocycloalkenyl, heterobicycloalkyl, heterobicycloalkenyl, heteropolycycloalkyl, heteropolycycloalkenyl, and the like unless indicated otherwise. Heterocycloalkyl refers to cycloalkyl groups containing one or more heteroatoms (O, S, or N) within the ring. Heterocycloalkenyl as used herein refers to cycloalkenyl groups containing one or more heteroatoms (O, S or N) within the ring. Heterobicycloalkyl refers to bicycloalkyl groups containing one or more heteroatoms (O, S or N) within a ring. Heterobicycloalkenyl as used herein refers to bicycloalkenyl groups containing one or more heteroatoms (O, S or N) within a ring. A heterocycle can refer to, for example, a saturated or partially unsaturated 4- to 12 or 4-10-membered ring structure, including bridged or fused rings, and whose ring structures include one to three heteroatoms, such as nitrogen, oxygen, and sulfur. Where possible, heterocyclic rings may be linked to the adjacent radical through carbon or nitrogen. Examples of heterocyclic groups include, but are not limited to, pyrrolidine, piperidine, morpholine, thiomorpholine, piperazine, oxetane, azetidine, tetrahydrofuran or dihydrofuran etc.

Cycloalkyl, cycloalkenyl, heterocyclic, groups also include groups similar to those described above for each of these respective categories, but which are substituted with one or more oxo moieties.

The term "aryl", as used herein, refers to mono- or polycyclic aromatic carbocyclic ring systems. A polycyclic aryl is a polycyclic ring system that comprises at least one aromatic ring. Polycyclic aryls can comprise fused rings, covalently attached rings or a combination thereof. The term "aryl" embraces aromatic radicals, such as, phenyl, naphthyl, indenyl, tetrahydronaphthyl, and indanyl. An aryl group may be substituted or unsubstituted. In some embodiments, the aryl is a C₄-C₁₀ aryl. Examples of optionally substituted aryl are phenyl, substituted phenyl, napthyl and substituted naphthyl.

The term "heteroaryl", as used herein, refers to aromatic carbocyclic groups containing one or more heteroatoms (O, S, or N) within a ring. A heteroaryl group, unless indicated otherwise, can be monocyclic or polycyclic. A heteroaryl group may additionally be substituted or unsubstituted. The heteroaryl groups of this disclosure can also include ring systems substituted with one or more oxo moieties. A polycyclic heteroaryl can comprise fused rings, covalently attached rings or a combination thereof. A polycyclic heteroaryl is a polycyclic ring system that comprises at least one aromatic ring containing one or more heteroatoms within a ring. Examples of heteroaryl groups include, but are not limited to, pyridinyl, pyridazinyl, imidazolyl, pyrimidinyl, pyrazolyl, triazolyl, pyrazinyl, quinolyl, isoquinolyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, triazinyl, isoindolyl, purinyl, oxadiazolyl, thiadiazolyl, furazanyl, benzofurazanyl, benzothiophenyl, benzotriazolyl, benzothiazolyl, benzoxazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl, thiazolopyridinyl, oxazolopyridinyl and azaindolyl. The foregoing heteroaryl groups may be C-attached or heteroatom-attached (where such is possible). For instance, a group derived from pyrrole may be pyrrol-1-yl (N-attached), pyrrol-2-yl (C-attached), or pyrrol-3-yl (C-attached). In some embodiments, the heteroaryl is 4- to 12-membered heteroaryl. In yet other embodiments, the heteroaryl is a mono or bicyclic 4- to 10-membered heteroaryl.

The term "substituted" refers to substitution by independent replacement of one, two, or three or more of the hydrogen atoms with substituents including, but not limited to, and unless indicated otherwise, -C₁-C₁₂ alkyl, -C₂-C₁₂ alkenyl, -C₂-C₁₂ alkynyl, -C₃-C₁₂ cycloalkyl, -C₃-C₁₂ cycloalkenyl, C₃-C₁₂ cycloalkynyl, -heterocyclic, -F, -Cl, -Br, -I, -OH, - NO₂, -N₃, -CN, -NH₂, oxo, thioxo, -NHRₓ, -NRₓRₓ, dialkylamino, -diarylamino, - diheteroarylamino, -ORₓ, -C(O)R_{y}, -C(O)C(O)R_{y}, -OCO₂R_{y}, -OC(O)R_{y}, OC(O)C(O)R_{y}, - NHC(O)R_{y}, -NHCO₂R_{y}, -NHC(O)C(O)R_{y}, NHC(S)NH₂, -NHC(S)NHRₓ, -NHC(NH)NH₂, - NHC(NH)NHRₓ, -NHC(NH)Rₓ, -C(NH)NHRₓ, and (C=NRₓ)Rₓ; -NRxC(O)Rₓ, - NRₓC(O)N(Rₓ)₂, -NRxCO₂R_{y}, -NRxC(O)C(O)R_{y}, -NRₓC(S)NH₂, -NRₓC(S)NHRₓ, - NRₓC(NH)NH₂, -NRₓC(NH)NHRₓ, -NRₓC(NH)Rₓ, -C(NRx)NHRₓ -S(O)R_{y}, -NHSO₂Rₓ, - CH₂NH₂, -CH₂SO₂CH₃, -aryl, -arylalkyl, -heteroaryl, -heteroarylalkyl, -heterocycloalkyl, -C₃-C₁₂ cycloalkyl, -polyalkoxyalkyl, -polyalkoxy, -methoxymethoxy, -methoxyethoxy, -SH, -S-Rₓ, or -methylthiomethyl, wherein Rₓ is selected from the group consisting of hydrogen, -C₁-C₁₂ alkyl, -C₂-C₁₂ alkenyl, -C₂-C₁₂ alkynyl, -C₃-C₁₂ cycloalkyl, -aryl, -heteroaryl and - heterocyclic and -R_{y} is selected from the group consisting of hydrogen, -C₁-C₁₂ alkyl, -C₂-C₁₂ alkenyl, -C₂-C₁₂ alkynyl, -C₃-C₁₂ cycloalkyl, -aryl, -heteroaryl, -heterocyclic, -NH₂, -NH-C₁-C₁₂ alkyl, -NH-C₂-C₁₂ alkenyl, -NH-C₂-C₁₂-alkynyl, -NH-C₃-C₁₂ cycloalkyl, -NH-aryl, -NH-heteroaryl and -NH-heterocyclic. It is understood that the aryls, heteroaryls, alkyls, and the like can be further substituted.

The terms "halo" or "halogen" as used herein refer to F, Cl, Br, or I.

The term "haloalkyl" as used herein refers to an alkyl group having 1 to (2n+1) substituent(s) independently selected from F, Cl, Br or I, where n is the maximum number of carbon atoms in the alkyl group. It will be understood that haloalkyl is a specific example of an optionally substituted alkyl.

The terms "hydroxy" and "hydroxyl" as used herein refers to the radical -OH.

As will be understood by the skilled artisan, "H" is the symbol for hydrogen, "N" is the symbol for nitrogen, "S" is the symbol for sulfur, "O" is the symbol for oxygen. "Me" is an abbreviation for methyl.

The compounds of the disclosure may contain one or more chiral centers and, therefore, exist as stereoisomers. The term "stereoisomers" when used herein consist of all enantiomers or diastereomers. These compounds may be designated by the symbols "(+)," "(-)," "*R*" or "*S*," depending on the configuration of substituents around the stereogenic carbon atom, but the skilled artisan will recognize that a structure may denote a chiral center implicitly. The present disclosure encompasses various stereoisomers of these compounds and mixtures thereof. Mixtures of enantiomers or diastereomers may be designated "(±)" in nomenclature, but the skilled artisan will recognize that a structure may denote a chiral center implicitly.

The compounds of the disclosure may contain one or more double bonds and, therefore, exist as geometric isomers resulting from the arrangement of substituents around a carbon-carbon double bond. The symbol 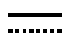 denotes a bond that may be a single, double or triple bond as described herein. Substituents around a carbon-carbon double bond are designated as being in the "*Z*" or "*E*" configuration wherein the terms "*Z*" and "*E*" are used in accordance with IUPAC standards. Unless otherwise specified, structures depicting double bonds encompass both the "*E*" and "*Z*" isomers. Substituents around a carbon-carbon double bond alternatively can be referred to as "cis" or "trans," where "cis" represents substituents on the same side of the double bond and "trans" represents substituents on opposite sides of the double bond.

Compounds of the disclosure may contain a carbocyclic or heterocyclic ring and therefore, exist as geometric isomers resulting from the arrangement of substituents around the ring. The arrangement of substituents around a carbocyclic or heterocyclic ring are designated as being in the "*Z*" or "*E*" configuration wherein the terms "*Z*" and "*E*" are used in accordance with IUPAC standards. Unless otherwise specified, structures depicting carbocyclic or heterocyclic rings encompass both "*Z*" and "*E*" isomers. Substituents around a carbocyclic or heterocyclic ring may also be referred to as "cis" or "trans", where the term "cis" represents substituents on the same side of the plane of the ring and the term "trans" represents substituents on opposite sides of the plane of the ring. Mixtures of compounds wherein the substituents are disposed on both the same and opposite sides of plane of the ring are designated "cis/trans."

Individual enantiomers and diasterisomers of compounds of the present disclosure can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, (3) direct separation of the mixture of optical enantiomers on chiral liquid chromatographic columns or (4) kinetic resolution using stereoselective chemical or enzymatic reagents. Racemic mixtures can also be resolved into their component enantiomers by well known methods, such as chiral-phase liquid chromatography or crystallizing the compound in a chiral solvent. Stereoselective syntheses, a chemical or enzymatic reaction in which a single reactant forms an unequal mixture of stereoisomers during the creation of a new stereocenter or during the transformation of a pre-existing one, are well known in the art. Stereoselective syntheses encompass both enantio- and diastereoselective transformations, and may involve the use of chiral auxiliaries. For examples, see Carreira and Kvaerno, Classics in Stereoselective Synthesis, Wiley-VCH: Weinheim, 2009. Where a particular compound is described or depicted, it is intended to encompass that chemical structure as well as tautomers of that structure.

The term "enantiomerically pure" means a stereomerically pure composition of a compound. For example, a stereochemically pure composition is a composition that is free or substantially free of other stereoisomers of that compound. In another example, for a compound having one chiral center, an enantiomerically pure composition of the compound is free or substantially free of the other enantiomer. In yet another example, for a compound having two chiral centers, an enantiomerically pure composition is free or substantially free of the other diastereomers.

Where a particular stereochemistry is described or depicted it is intended to mean that a particular enantiomer is present in excess relative to the other enantiomer. A compound has an R-configuration at a specific position when it is present in excess compared to the compound having an S-configuration at that position. A compound has an *S-*configuration at a specific position when it is present in excess compared to the compound having an *R*-configuration at that position.

The compounds disclosed herein can exist in solvated as well as unsolvated forms with pharmaceutically acceptable solvents such as water, ethanol, and the like, and it is intended that the disclosure embrace both solvated and unsolvated forms. In one embodiment, the compound is amorphous. In one embodiment, the compound is a single polymorph. In another embodiment, the compound is a mixture of polymorphs. In another embodiment, the compound is in a crystalline form.

The disclosure also embraces isotopically labeled compounds of the disclosure which are identical to those recited herein, except that one or more atoms are replaced by an atom having an atomic mass or mass number different from the atomic mass or mass number usually found in nature. Examples of isotopes that can be incorporated into compounds of the disclosure include isotopes of hydrogen, carbon, nitrogen, oxygen, phosphorus, sulfur, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. For example, a compound of the disclosure may have one or more H atom replaced with deuterium.

For example, a disclosed compound may have one or more H atoms replaced with deuterium. It will be recognized that some variation of natural isotopic abundance occurs in a synthesized compound depending upon the origin of chemical materials used in the synthesis. Thus, a preparation of a disclosed compound will inherently contain small amounts of deuterated isotopologues. The concentration of naturally abundant stable hydrogen and carbon isotopes, notwithstanding this variation, is small and immaterial as compared to the degree of stable isotopic substitution of compounds of this disclosure.

In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Also unless otherwise stated, when a position is designated specifically as "D" or "deuterium", the position is understood to have deuterium at an abundance that is at least 3000 times greater than the natural abundance of deuterium, which is 0.015% (i.e., at least 45% incorporation of deuterium).

The term "isotopic enrichment factor" as used herein means the ratio between the isotopic abundance and the natural abundance of a specified isotope.

In other embodiments, a disclosed compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The term "isotopologue" refers to a species in which the chemical structure differs from a specific compound of this disclosure only in the isotopic composition thereof.

The term "compound," when referring to a compound of this disclosure, refers to a collection of molecules having an identical chemical structure, except that there may be isotopic variation among the constituent atoms of the molecules. Thus, it will be clear to those of skill in the art that a compound represented by a particular chemical structure containing indicated deuterium atoms, will also contain lesser amounts of isotopologues having hydrogen atoms at one or more of the designated deuterium positions in that structure. The relative amount of such isotopologues in a compound of this disclosure will depend upon a number of factors including the isotopic purity of deuterated reagents used to make the compound and the efficiency of incorporation of deuterium in the various synthesis steps used to prepare the compound. However, as set forth above the relative amount of such isotopologues in total will be less than 49.9% of the compound. In other embodiments, the relative amount of such isotopologues in total will be less than 47.5%, less than 40%, less than 32.5%, less than 25%, less than 17.5%, less than 10%, less than 5%, less than 3%, less than 1%, or less than 0.5% of the compound.

Certain isotopically-labeled disclosed compounds (*e.g.*, those labeled with ³H and ¹⁴C) are useful in compound and/or substrate tissue distribution assays. Tritiated (*i.e*., ³H) and carbon-14 (*i.e*., ¹⁴C) isotopes are particularly suitable for their ease of preparation and detectability. Further, substitution with heavier isotopes such as deuterium (*i.e*., ²H) may afford certain therapeutic advantages resulting from greater metabolic stability (*e.g*., increased *in vivo* half-life or reduced dosage requirements) and hence may be suitable in some circumstances. Isotopically labeled compounds of the disclosure can generally be prepared by following procedures analogous to those disclosed in the examples herein by substituting an isotopically labeled reagent for a non-isotopically labeled reagent.

The disclosure additionally encompasses embodiments wherein one or more of the nitrogen atoms in a disclosed compound are oxidized to N-oxide.

Representative and exemplary synthetic routes for the preparation of compounds described herein are shown in the schemes below and throughout the Examples section. As will be understood by the skilled artisan, diastereomers can be separated from the reaction mixture using column chromatography.

Compounds of the disclosure can also be prepared using methods described in the literature, including, but not limited to, J. Med. Chem. 2011, 54(13), 4350-64; Russian Journal of Organic Chemistry 2011, 47(8), 1199-1203; U.S. Patent Application Publication No. 2009/0036451 A1; WO2008/046072 A2, and U.S. Patent No. 4,336,264.

As discussed above, the disclosure encompasses to a method of enhancing (e.g., increasing) CFTR activity in a subject (e.g., a subject suffering from any one or more of the conditions described herein) comprising administering a first compound of Formula Ia, Ib, Ic or Id, a second compound of Formula II, and a third compound of Formula III or IV, as disclosed herein, in an effective amount. The disclosure also encompasses a method of treating a patient suffering from a condition associated with CFTR activity comprising administering to said patient an effective amount of a first compound of Formula Ia, Ib, Ic or Id, a second compound of Formula II, and a third compound of Formula III or IV, as disclosed herein. In certain embodiments, the disease is cystic fibrosis.

"Treating" or "treatment" includes preventing or delaying the onset of the symptoms, complications, or biochemical indicia of a disease, alleviating or ameliorating the symptoms or arresting or inhibiting further development of the disease, condition, or disorder. A "subject" is an animal to be treated or in need of treatment. A "patient" is a human subject in need of treatment.

An "effective amount" or "therapeutically effective amount" refers to that amount of an agent that is sufficient to achieve a desired and/or recited effect. In the context of a method of treatment, an "effective amount" or "therapeutically effective amount" of the therapeutic agent that is sufficient to ameliorate of one or more symptoms of a disorder and/or prevent advancement of a disorder, cause regression of the disorder and/or to achieve a desired effect.

The term "modulating" encompasses increasing, enhancing, inhibiting, decreasing, suppressing, and the like. The terms "increasing" and "enhancing" mean to cause a net gain by either direct or indirect means. As used herein, the terms "inhibiting" and "decreasing" encompass causing a net decrease by either direct or indirect means.

In some examples, CFTR activity is enhanced after administration of first compound of Formula Ia, Ib, Ic or Id, a second compound of Formula II, and a third compound of Formula III or IV, as disclosed herein, when there is an increase in the CFTR activity as compared to that in the absence of the administration of disclosed compounds. CFTR activity encompasses, for example, chloride channel activity of the CFTR, and/or other ion transport activity for example, HCO₃⁻ transport). In certain of these embodiments, the activity of one or more (e.g., one or two) mutant CFTRs (e.g., ΔF508, S549N, G542X, G551D, R117H, N1303K, W1282X, R553X, 621+1G>T, 1717-1G>A, 3849+10kbC>T, 2789+5G>A, 3120+1G>A, I507del, R1162X, 1898+1G>A, 3659delC, G85E, D1152H, R560T, R347P, 2184insA, A455E, R334W, Q493X, and 2184delA CFTR) is enhanced (e.g., increased). Contemplated patients may have a CFTR mutation(s) from one or more classes, such as without limitation, Class I CFTR mutations, Class II CFTR mutations, Class III CFTR mutations, Class IV CFTR mutations, Class V CFTR mutations, and Class VI mutations. Contemplated subject (e.g., human subject) CFTR genotypes include, without limitation, homozygote mutations (e.g., ΔF508 / ΔF508 and R117H / R117H) and compound heterozygote mutations (e.g., ΔF508 / G551D; ΔF508 / A455E; ΔF508 / G542X; Δ508F / W1204X; Δ508F / S549N; R553X / W1316X; W1282X/N1303K, 591Δ18 / E831X; F508del/R117H/ N1303K/ 3849+10kbC>T; Δ 303K/ 384 and DF508/G178R).

In certain embodiments, the mutation is a Class I mutation, e.g., a G542X; a Class III I mutation, e.g., a ΔF508 / G542X compound heterozygous mutation. In other embodiments, the mutation is a Class III mutation, e.g., a G551D; a Class III Class III mutation, e.g., a ΔF508 / G551D compound heterozygous mutation. In still other embodiments, the mutation is a Class V mutation, e.g., a A455E; Class III Class V mutation, e.g., a ΔF508 / A455E compound heterozygous mutation. Of the more than 1000 known mutations of the *CFTR* gene, ΔF508 is the most prevalent mutation of CFTR which results in misfolding of the protein and impaired trafficking from the endoplasmic reticulum to the apical membrane (Dormer et al. (2001), J. Cell Sci. 114, 4073-4081; http://www.genet.sickkids.on.ca/app). In certain embodiments, ΔF508 CFTR activity is enhanced (e.g., increased). In certain embodiments, ΔF508 CFTR activity and/or G542X CFTR activity and/or G551D CFTR activity and/or A455E CFTR activity is enhanced (e.g., increased). An enhancement of CFTR activity can be measured, for example, using literature described methods, including for example, Ussing chamber assays, patch clamp assays, and hBE Ieq assay (Devor et al. (2000), Am. J. Physiol. Cell Physiol. 279(2): C461-79; Dousmanis et al. (2002), J. Gen. Physiol. 119(6): 545-59; Bruscia et al. (2005), PNAS 103(8): 2965-2971).

In certain of these embodiments, the activity of one or more (e.g., one or two) mutant CFTRs (e.g., ΔF508, S549N, G542X, G551D, R117H, N1303K, W1282X, R553X, 621+1G>T, 1717-1G>A, 3849+10kbC>T, 2789+5G>A, 3120+1G>A, I507del, R1162X, 1898+1G>A, 3659delC, G85E, D1152H, R560T, R347P, 2184insA, A455E, R334W, Q493X, E56K, P67L, R74W, D110E, D110H, R117C, G178R, E193K, L206W, R347H, R352Q, A455E, S549R, G551S, D579G, S945L, S997F, F1052V, K1060T, A1067T, G1069R, R1070Q, R1070W, F1074L, G1244E, S1251N, S1255P, D1270N, G1349D, and 2184delA CFTR) is enhanced (e.g., increased). In certain embodiments, ΔF508 CFTR activity is enhanced (e.g., increased). In other embodiments, the activities of two mutant CFTRs (e.g., ΔF508 and G551D; ΔF508 and A455E; or G542X; Δ508F) are enhanced (e.g., increased).

As discussed above, the disclosure also encompasses a method of treating cystic fibrosis. The present disclosure can also be used to treat other conditions associated with CFTR activity, including conditions associated with deficient CFTR activity.

In some embodiments, the disclosure is directed to a method of treating a condition associated with deficient or decreased CFTR activity comprising administering an effective amount of a first compound of Formula Ia, Ib, Ic or Id, a second compound of Formula II, and a third compound of Formula III or IV, as disclosed herein. Non-limiting examples of conditions associated with deficient CFTR activity are cystic fibrosis, congenital bilateral absence of vas deferens (CBAVD), acute, recurrent, or chronic pancreatitis, disseminated bronchiectasis, asthma, allergic pulmonary aspergillosis, smoking-related lung diseases, such as chronic obstructive pulmonary disease (COPD), chronic sinusitis, dry eye disease, protein C deficiency, Aβ-lipoproteinemia, lysosomal storage disease, type 1 chylomicronemia, mild pulmonary disease, lipid processing deficiencies, type 1 hereditary angioedema, coagulation-fibrinolyis, hereditary hemochromatosis, CFTR-related metabolic syndrome, chronic bronchitis, constipation, pancreatic insufficiency, hereditary emphysema, and Sjogren's syndrome.

Further examples of CFTR modulators, for example CFTR correctors, contemplated herein include, e.g., (R)-8-methyl-2-(3-methylbenzo[b]thiophen-2-yl)-N-(methylsulfonyl)-5-(1-phenylethoxy)quinoline-4-carboxamide, (R)-8-methyl-2-(3-methylbenzofuran-2-yl)-N-(methylsulfonyl)-5-(1-phenylethoxy)quinoline-4-carboxamide, (R)-N-(cyclopropylsulfonyl)-8-methyl-2-(3-methylbenzo[b]thiophen-2-yl)-5-(1-phenylethoxy)quinoline-4-carboxamide, (R)-N-(cyclopropylsulfonyl)-8-methyl-2-(3-methylbenzofuran-2-yl)-5-(1-phenylethoxy)quinoline-4-carboxamide, (R)-N-((2-hydroxyethyl)sulfonyl)-8-methyl-2-(3-methylbenzo[b]thiophen-2-yl)-5-(1-phenylethoxy)quinoline-4-carboxamide, (R)-N-((2-hydroxyethyl)sulfonyl)-8-methyl-2-(3-methylbenzofuran-2-yl)-5-(1-phenylethoxy)quinoline-4-carboxamide, (R)-8-methyl-2-(3-methylbenzo[b]thiophen-2-yl)-N-(morpholinosulfonyl)-5-(1-phenylethoxy)quinoline-4-carboxamide, (R)-8-methyl-2-(3-methylbenzofuran-2-yl)-N-(morpholinosulfonyl)-5-(1-phenylethoxy)quinoline-4-carboxamide, (R)-8-methyl-2-(3-methylbenzo[b]thiophen-2-yl)-5-(1-phenylethoxy)-N-(phenylsulfonyl)quinoline-4-carboxamide, (R)-8-methyl-2-(3-methylbenzofuran-2-yl)-5-(1-phenylethoxy)-N-(phenylsulfonyl)quinoline-4-carboxamide, (R)-N-((6-aminopyridin-2-yl)sulfonyl)-8-methyl-2-(3-methylbenzo[b]thiophen-2-yl)-5-(1-phenylethoxy)quinoline-4-carboxamide, (R)-N-((6-aminopyridin-2-yl)sulfonyl)-8-methyl-2-(3-methylbenzofuran-2-yl)-5-(1-phenylethoxy)quinoline-4-carboxamide, (R)-N-((3-aminophenyl)sulfonyl)-8-methyl-2-(3-methylbenzo[b]thiophen-2-yl)-5-(1-phenylethoxy)quinoline-4-carboxamide, (R)-N-((3-aminophenyl)sulfonyl)-8-methyl-2-(3-methylbenzofuran-2-yl)-5-(1-phenylethoxy)quinoline-4-carboxamide, N-(((S)-3-aminopyrrolidin-1-yl)sulfonyl)-8-methyl-2-(3-methylbenzo[b]thiophen-2-yl)-5-((R)-1-phenylethoxy)quinoline-4-carboxamide, N-(((S)-3-aminopyrrolidin-1-yl)sulfonyl)-8-methyl-2-(3-methylbenzofuran-2-yl)-5-((R)-1-phenylethoxy)quinoline-4-carboxamide, (R)-N-((2-aminothiazol-5-yl)sulfonyl)-8-methyl-2-(3-methylbenzo[b]thiophen-2-yl)-5-(1-phenylethoxy)quinoline-4-carboxamide, (R)-N-((2-aminothiazol-5-yl)sulfonyl)-8-methyl-2-(3-methylbenzofuran-2-yl)-5-(1-phenylethoxy)quinoline-4-carboxamide, (R)-N-((1H-pyrazol-5-yl)sulfonyl)-8-methyl-2-(3-methylbenzo[b]thiophen-2-yl)-5-(1-phenylethoxy)quinoline-4-carboxamide, (R)-N-((1H-pyrazol-5-yl)sulfonyl)-8-methyl-2-(3-methylbenzofuran-2-yl)-5-(1-phenylethoxy)quinoline-4-carboxamide, (R)-8-methyl-2-(3-methylbenzo[b]thiophen-2-yl)-N-(methylsulfonyl)-5-(1-(tetrahydro-2H-pyran-4-yl)ethoxy)quinoline-4-carboxamide, (R)-8-methyl-2-(3-methylbenzofuran-2-yl)-N-(methylsulfonyl)-5-(1-(tetrahydro-2H-pyran-4-yl)ethoxy)quinoline-4-carboxamide, N-(cyclopropylsulfonyl)-8-methyl-2-(3-methylbenzo[b]thiophen-2-yl)-5-((2,2,6,6-tetramethyltetrahydro-2H-pyran-4-yl)methoxy)quinoline-4-carboxamide, N-(cyclopropylsulfonyl)-8-methyl-2-(3-methylbenzofuran-2-yl)-5-((2,2,6,6-tetramethyltetrahydro-2H-pyran-4-yl)methoxy)quinoline-4-carboxamide, (R)-8-methyl-2-(3-methylbenzo[b]thiophen-2-yl)-N-(morpholinosulfonyl)-5-(1-(tetrahydro-2H-pyran-4-yl)ethoxy)quinoline-4-carboxamide, 8-methyl-2-(3-methylbenzofuran-2-yl)-N-(morpholinosulfonyl)-5-((2,2,6,6-tetramethyltetrahydro-2H-pyran-4-yl)methoxy)quinoline-4-carboxamide, 8-methyl-2-(3-methylbenzofuran-2-yl)-N-(methylsulfonyl)-5-((2,2,6,6-tetramethyltetrahydro-2H-pyran-4-yl)methoxy)quinoline-4-carboxamide, 8-methyl-2-(3-methylbenzo[b]thiophen-2-yl)-N-(methylsulfonyl)-5-((2,2,6,6-tetramethyltetrahydro-2H-pyran-4-yl)methoxy)quinoline-4-carboxamide, N-((2-hydroxyethyl)sulfonyl)-8-methyl-2-(3-methylbenzofuran-2-yl)-5-((2,2,6,6-tetramethyltetrahydro-2H-pyran-4-yl)methoxy)quinoline-4-carboxamide, N-((2-hydroxyethyl)sulfonyl)-8-methyl-2-(3-methylbenzo[b]thiophen-2-yl)-5-((2,2,6,6-tetramethyltetrahydro-2H-pyran-4-yl)methoxy)quinoline-4-carboxamide, 8-methyl-2-(3-methylbenzo[b]thiophen-2-yl)-N-(morpholinosulfonyl)-5-((2,2,6,6-tetramethyltetrahydro-2H-pyran-4-yl)methoxy)quinoline-4-carboxamide, 8-methyl-2-(3-methylbenzofuran-2-yl)-N-(phenylsulfonyl)-5-((2,2,6,6-tetramethyltetrahydro-2H-pyran-4-yl)methoxy)quinoline-4-carboxamide, 8-methyl-2-(3-methylbenzo[b]thiophen-2-yl)-N-(phenylsulfonyl)-5-((2,2,6,6-tetramethyltetrahydro-2H-pyran-4-yl)methoxy)quinoline-4-carboxamide, N-((3-aminophenyl)sulfonyl)-8-methyl-2-(3-methylbenzofuran-2-yl)-5-((2,2,6,6-tetramethyltetrahydro-2H-pyran-4-yl)methoxy)quinoline-4-carboxamide, N-((3-aminophenyl)sulfonyl)-8-methyl-2-(3-methylbenzo[b]thiophen-2-yl)-5-((2,2,6,6-tetramethyltetrahydro-2H-pyran-4-yl)methoxy)quinoline-4-carboxamide, N-((6-aminopyridin-2-yl)sulfonyl)-8-methyl-2-(3-methylbenzofuran-2-yl)-5-((2,2,6,6-tetramethyltetrahydro-2H-pyran-4-yl)methoxy)quinoline-4-carboxamide, N-((6-aminopyridin-2-yl)sulfonyl)-8-methyl-2-(3-methylbenzo[b]thiophen-2-yl)-5-((2,2,6,6-tetramethyltetrahydro-2H-pyran-4-yl)methoxy)quinoline-4-carboxamide, (S)-N-((3-aminopyrrolidin-1-yl)sulfonyl)-8-methyl-2-(3-methylbenzofuran-2-yl)-5-((2,2,6,6-tetramethyltetrahydro-2H-pyran-4-yl)methoxy)quinoline-4-carboxamide, (S)-N-((3-aminopyrrolidin-1-yl)sulfonyl)-8-methyl-2-(3-methylbenzo[b]thiophen-2-yl)-5-((2,2,6,6-tetramethyltetrahydro-2H-pyran-4-yl)methoxy)quinoline-4-carboxamide, N-((2-aminothiazol-5-yl)sulfonyl)-8-methyl-2-(3-methylbenzofuran-2-yl)-5-((2,2,6,6-tetramethyltetrahydro-2H-pyran-4-yl)methoxy)quinoline-4-carboxamide, N-((2-aminothiazol-5-yl)sulfonyl)-8-methyl-2-(3-methylbenzo[b]thiophen-2-yl)-5-((2,2,6,6-tetramethyltetrahydro-2H-pyran-4-yl)methoxy)quinoline-4-carboxamide, N-((1H-pyrazol-5-yl)sulfonyl)-8-methyl-2-(3-methylbenzofuran-2-yl)-5-((2,2,6,6-tetramethyltetrahydro-2H-pyran-4-yl)methoxy)quinoline-4-carboxamide, N-((1H-pyrazol-5-yl)sulfonyl)-8-methyl-2-(3-methylbenzo[b]thiophen-2-yl)-5-((2,2,6,6-tetramethyltetrahydro-2H-pyran-4-yl)methoxy)quinoline-4-carboxamide, (R)-N-(cyclopropylsulfonyl)-8-methyl-2-(3-methylbenzofuran-2-yl)-5-(1-(tetrahydro-2H-pyran-4-yl)ethoxy)quinoline-4-carboxamide, (R)-N-(cyclopropylsulfonyl)-8-methyl-2-(3-methylbenzo[b]thiophen-2-yl)-5-(1-(tetrahydro-2H-pyran-4-yl)ethoxy)quinoline-4-carboxamide, (R)-N-((2-hydroxyethyl)sulfonyl)-8-methyl-2-(3-methylbenzofuran-2-yl)-5-(1-(tetrahydro-2H-pyran-4-yl)ethoxy)quinoline-4-carboxamide, (R)-N-((2-hydroxyethyl)sulfonyl)-8-methyl-2-(3-methylbenzo[b]thiophen-2-yl)-5-(1-(tetrahydro-2H-pyran-4-yl)ethoxy)quinoline-4-carboxamide, (R)-8-methyl-2-(3-methylbenzofuran-2-yl)-N-(morpholinosulfonyl)-5-(1-(tetrahydro-2H-pyran-4-yl)ethoxy)quinoline-4-carboxamide, (R)-8-methyl-2-(3-methylbenzo[b]thiophen-2-yl)-N-(morpholinosulfonyl)-5-(1-(tetrahydro-2H-pyran-4-yl)ethoxy)quinoline-4-carboxamide, (R)-8-methyl-2-(3-methylbenzo[b]thiophen-2-yl)-N-(phenylsulfonyl)-5-(1-(tetrahydro-2H-pyran-4-yl)ethoxy)quinoline-4-carboxamide, (R)-N-((3-aminophenyl)sulfonyl)-8-methyl-2-(3-methylbenzofuran-2-yl)-5-(1-(tetrahydro-2H-pyran-4-yl)ethoxy)quinoline-4-carboxamide, (R)-N-((3-aminophenyl)sulfonyl)-8-methyl-2-(3-methylbenzo[b]thiophen-2-yl)-5-(1-(tetrahydro-2H-pyran-4-yl)ethoxy)quinoline-4-carboxamide, (R)-N-((6-aminopyridin-2-yl)sulfonyl)-8-methyl-2-(3-methylbenzofuran-2-yl)-5-(1-(tetrahydro-2H-pyran-4-yl)ethoxy)quinoline-4-carboxamide, (R)-N-((6-aminopyridin-2-yl)sulfonyl)-8-methyl-2-(3-methylbenzo[b]thiophen-2-yl)-5-(1-(tetrahydro-2H-pyran-4-yl)ethoxy)quinoline-4-carboxamide, N-(((S)-3-aminopyrrolidin-1-yl)sulfonyl)-8-methyl-2-(3-methylbenzofuran-2-yl)-5-((R)-1-(tetrahydro-2H-pyran-4-yl)ethoxy)quinoline-4-carboxamide, N-(((S)-3-aminopyrrolidin-1-yl)sulfonyl)-8-methyl-2-(3-methylbenzo[b]thiophen-2-yl)-5-((R)-1-(tetrahydro-2H-pyran-4-yl)ethoxy)quinoline-4-carboxamide, (R)-N-((2-aminothiazol-5-yl)sulfonyl)-8-methyl-2-(3-methylbenzofuran-2-yl)-5-(1-(tetrahydro-2H-pyran-4-yl)ethoxy)quinoline-4-carboxamide, (R)-N-((2-aminothiazol-5-yl)sulfonyl)-8-methyl-2-(3-methylbenzo[b]thiophen-2-yl)-5-(1-(tetrahydro-2H-pyran-4-yl)ethoxy)quinoline-4-carboxamide, (R)-N-((1H-pyrazol-5-yl)sulfonyl)-8-methyl-2-(3-methylbenzofuran-2-yl)-5-(1-(tetrahydro-2H-pyran-4-yl)ethoxy)quinoline-4-carboxamide, (R)-N-((1H-pyrazol-5-yl)sulfonyl)-8-methyl-2-(3-methylbenzo[b]thiophen-2-yl)-5-(1-(tetrahydro-2H-pyran-4-yl)ethoxy)quinoline-4-carboxamide, and pharmaceutically acceptable salts and/or stereoisomers thereof.

In some embodiments, disclosed methods of treatment further comprise administering an additional therapeutic agent. For example, in an embodiment, provided herein is a method of administering an effective amount of a first compound of Formula Ia, Ib, Ic or Id, a second compound of Formula II, and a third compound of Formula III or IV, and an additional therapeutic agent. Additional therapeutic agents include, for example, mucolytic agents, bronchodilators, antibiotics, anti-infective agents, anti-inflammatory agents, ion channel modulating agents, therapeutic agents used in gene therapy, CFTR amplifiers, CFTR correctors, or other agents that modulates CFTR activity. In some embodiments, the additional therapeutic agent is a CFTR amplifier or a CFTR corrector. Non-limiting examples of CFTR correctors include (3-(6-(1-(2,2-difluorobenzo[d][1,3]dioxol-5-yl)cyclopropanecarboxamido)-3-methylpyridin-2-yl)benzoic acid (lumacaftor), deuterated lumacaftor, ((R)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropane-1-carboxamide (tezacaftor), deuterated tezacaftor, VX-152, VX-440, VX-445, VX-659, GLPG2222, GLPG2851, GLPG2737, GLPG3221 and VX-983, and compounds described in, e.g., WO2017/062581. Non-limiting examples of modulators include QBW-251, QR-010, NB-124, riociquat, SPX-101, and compounds described in, e.g., WO2014/144860, 2014/176553, WO2014/045283; WO2014/081821, WO2014/081820, WO2014/152213; WO2014/160440, WO2014/160478, US2014027933; WO2014/0228376, WO2013/038390, WO2011/113894, WO2013/038386; and WO2014/180562, of which the disclosed modulators in those publications are contemplated as an additional therapeutic agent.

Non-limiting examples of anti-inflammatory agents include N6022 (3-(5-(4-(1H-imidazol-1-yl) phenyl)-1-(4-carbamoyl-2-methylphenyl)-¹H-pyrrol-2-yl) propanoic acid), CTX-4430, N1861, N1785, and N91115. In certain of these embodiments, the CFTR modulator is an agent that enhances read-through of stop codons (e.g., NB124 or ataluren).

In certain embodiments, the subject's CFTR genotype includes, without limitation, one or more Class I CFTR mutations, one or more Class II CFTR mutations, one or more Class III CFTR mutations, one or more Class IV CFTR mutations, or one or more Class V CFTR mutations, or one or more Class VI CFTR mutations. In certain embodiments, the subject's CFTR genotype includes, without limitation, one or more homozygote mutations (e.g., ΔF508 / ΔF508 or R117H / R117H) and/or one or more compound heterozygote mutations (e.g., ΔF508 / G551D; ΔF508 / A455E; ΔF508 / G542X; Δ508F / W1204X; Δ508F / S549N; R553X / W1316X; W1282X/N1303K; F508del/R17H; N1303K/ 3849+10kbC>T; ΔF508/R334W; DF508/G178R; and 591Δ18 / E831X). In certain embodiments, the subject's CFTR genotype includes a Class I mutation, e.g., a G542X Class I mutation, e.g., a ΔF508 / G542X compound heterozygous mutation. In other embodiments, the subject's CFTR genotype includes a Class III mutation, e.g., a G551D Class III mutation, e.g., a ΔF508 / G551D compound heterozygous mutation. In still other embodiments, the subject's CFTR genotype includes a Class V mutation, e.g., a A455E Class V mutation, e.g., a ΔF508 / A455E compound heterozygous mutation. In certain embodiments, ΔF508 CFTR activity and/or G542X CFTR activity and/or GSS 1D CFTR activity and/or A455E activity is enhanced (e.g., increased). In certain embodiments, the enhancement in activity (e.g., increase in activity) provided by the combination of disclosed compounds is greater than additive when compared to the enhancement in activity provided by each therapeutic component individually.

| **Class** | **Effect on CFTR protein** | **Example of mutation** |
|---|---|---|
| I | Shortened protein | W1282X Instead of inserting the amino acid tryptophan (W), the protein sequence is prematurely stopped (indicated by an X). |
| II | Protein fails to reach cell membrane | ΔF508 A phenylalanine amino acid (F) is deleted |
| III | Channel cannot be regulated properly | G551D A "missense" mutation: instead of a glycine amino acid (G), aspartate (D) is added |
| IV | Reduced chloride conductance | R117H Missense |
| V | Reduced due to incorrect splicing of gene | 3120+1G>A Splice-site mutation in gene intron 16 |
| VI | Reduced due to protein instability | N287Y a A ->T at 991 |

| **Genotype** | **Description** | **Possible Symptoms** |
|---|---|---|
| Δ508F / Δ508F | homozygote | Severe lung disease, pancreatic insufficient |
| R117H / R117H | homozygote | Congenital bilateral absence of the vas deferens, |
| | | No lung or pancreas disease |
| WT / Δ508F | heterozygote | Unaffected |
| WT / 3120+1 G>A | heterozygote | Unaffected |
| Δ508F / W1204X | compound heterozygote | No lung disease, pancreatic insufficient |
| R553X and W1316X | compound heterozygote | Mild lung disease, pancreatic insufficient |
| 591Δ18 / E831X | compound heterozygote | No lung or pancreas disease, nasal polyps |

For example, provided herein is a method of treating a patient having one or more of the following mutations in the CFTR gene: ΔF508, G542X, G1244E, G1349D, G178R, G551S, S1251N, S1255P, S549N, S549R, G970R, orR117H, and/or e.g., a patient with one or two copies of the F508del mutation, or one copy of the ΔF508 mutation and a second mutation that results in a gating effect in the CFTR protein (e.g., a patient that is heterozygous for ΔF508 and G542X or G551D mutation), a patient with one copy of the ΔF508 mutation and a second mutation that results in residual CFTR activity , or a patient with one copy of the ΔF508 mutation and a second mutation that results in residual CFTR activity, comprising administering an effective amount of a disclosed compound. As described herein, such exemplary methods (e.g., of a patient having one or mutations such as those described above) may include, for example, administering to such patient a combination therapy, e.g., administering (simultaneously or sequentially) an effective amount of a first compound of Formula Ia, Ib, Ic or Id, a second compound of Formula II, and a third compound of Formula III or IV, to said patient. Such administration may result, for example, in increased chloride transport in human bronchial epithelial cells with e.g., one or two copies of mutations, e.g, ΔF508 mutation, as compared to administration of a disclosed compound alone.

The phrase "combination therapy," as used herein, refers to an embodiment where a patient is co-administered an effective amount of a first compound of Formula Ia, Ib, Ic or Id, a second compound of Formula II, and a third compound of Formula III or IV, as described herein, an optionally one or more additional CFTR modulators as described anywhere herein, as part of a specific treatment regimen intended to provide the beneficial effect from the co-action of these therapeutic agents. For example, a beneficial effect of a combination may include, but is not limited to, pharmacokinetic or pharmacodynamic co-action resulting from the combination of therapeutic agents. For example, administration of a first compound of Formula Ia, Ib, Ic or Id, a second compound of Formula II, and a third compound of Formula III or IV, may result in a level of function, e.g., as measured by chloride activity in HBE cells or patients that have a ΔF508 mutation, that achieves clinical improvement (or better) as compared to the chloride activity level in cells or patients with a ΔF508 mutation receiving a disclosed compound alone; or for example, administration of a first compound of Formula Ia, Ib, Ic or Id, a second compound of Formula II, and a third compound of Formula III or IV, may result in a level of function (e.g., as measured by chloride activity in HBE cells or patients that have a G542X mutation, that achieves clinical improvement (or better) as compared to the chloride activity level at e.g., 50% or more of wild type cells. Administration of disclosed therapeutic agents in combination typically is carried out over a defined time period (usually a day, days, weeks, months or years depending upon the combination selected). Combination therapy is intended to embrace administration of multiple therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single tablet or capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, inhalational routes, intravenous routes, intramuscular routes, and direct absorption through mucous membrane tissues. The therapeutic agents can be administered by the same route or by different routes. For example, a first therapeutic agent of the combination selected may be administered by intravenous injection or inhalation or nebulizer while the other therapeutic agents of the combination may be administered orally. Alternatively, for example, all therapeutic agents may be administered orally or all therapeutic agents may be administered by intravenous injection, inhalation or nebulization.

Combination therapy also can embrace the administration of the therapeutic agents as described herein in further combination with other biologically active ingredients and non-drug therapies. Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by a day, days or even weeks.

The components of a disclosed combination may be administered to a patient simultaneously or sequentially. It will be appreciated that the components may be present in the same pharmaceutically acceptable carrier and, therefore, are administered simultaneously. Alternatively, the active ingredients may be present in separate pharmaceutical carriers, such as, conventional oral dosage forms, that can be administered either simultaneously or sequentially.

The term "pharmaceutically acceptable salt(s)" as used herein refers to salts of acidic or basic groups that may be present in a disclosed compound used in disclosed compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including, but not limited to, malate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, *p-*toluenesulfonate and pamoate (i.e., 1,1'-methylene-*bis*-(2-hydroxy-3-naphthoate)) salts. Compounds included in the present compositions that are acidic in nature are capable of forming base salts with various pharmacologically acceptable cations. Examples of such salts include alkali metal or alkaline earth metal salts, particularly calcium, magnesium, sodium, lithium, zinc, potassium, and iron salts. Compounds included in the present compositions that include a basic or acidic moiety may also form pharmaceutically acceptable salts with various amino acids. The compounds of the disclosure may contain both acidic and basic groups; for example, one amino and one carboxylic acid group. In such a case, the compound can exist as an acid addition salt, a zwitterion, or a base salt.

Also included in the present disclosure are methods that include administering prodrugs of the compounds described herein, for example, prodrugs of a compound of Formula Ia, Ib, Ic or Id, II, or III, or a pharmaceutical composition thereof or method of use of the prodrug.

The term "prodrug" refers to compounds that are transformed *in vivo* to yield a disclosed compound or a pharmaceutically acceptable salt, hydrate or solvate of the compound. The transformation may occur by various mechanisms (such as by esterase, amidase, phosphatase, oxidative and or reductive metabolism) in various locations (such as in the intestinal lumen or upon transit of the intestine, blood or liver). Prodrugs are well known in the art (for example, see Rautio, Kumpulainen, et al., Nature Reviews Drug Discovery 2008, 7, 255). For example, if a compound of the disclosure or a pharmaceutically acceptable salt, hydrate or solvate of the compound contains a carboxylic acid functional group, a prodrug can comprise an ester formed by the replacement of the hydrogen atom of the acid group with a group such as (C₁₋₈) alkyl, (C₂₋₁₂) alkylcarbonyloxymethyl, 1-(alkylcarbonyloxy)ethyl having from 4 to 9 carbon atoms, 1-methyl-1-(alkylcarbonyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁₋₂)alkylamino(C₂₋₃)alkyl (such as β-dimethylaminoethyl), carbamoyl-(C₁₋₂)alkyl, N,N-di(C₁₋₂)alkylcarbamoyl-(C₁₋₂)alkyl and piperidino-, pyrrolidino- or morpholino(C₂₋₃)alkyl.

Similarly, if a compound of the disclosure contains an alcohol functional group, a prodrug can be formed by the replacement of the hydrogen atom of the alcohol group with a group such as (C₁₋₆)alkylcarbonyloxymethyl, 1-((C₁₋₆)alkylcarbonyloxy)ethyl, 1-methyl-1-((C₁₋₆)alkylcarbonyloxy)ethyl (C₁₋₆)alkoxycarbonyloxymethyl, N-(C₁₋₆)alkoxycarbonylaminomethyl, succinoyl, (C₁₋₆)alkylcarbonyl, α-amino(C₁₋₄)alkylcarbonyl, arylalkylcarbonyl and α-aminoalkylcarbonyl, or α-aminoalkylcarbonyl-α-aminoalkylcarbonyl, where each α-aminoalkylcarbonyl group is independently selected from the naturally occurring L-amino acids, P(O)(OH)₂, -P(O)(O(C₁₋₆)alkyl)₂ or glycosyl (the radical resulting from the removal of a hydroxyl group of the hemiacetal form of a carbohydrate).

If a compound of the disclosure incorporates an amine functional group, a prodrug can be formed, for example, by creation of an amide or carbamate, an N-alkylcarbonyloxyalkyl derivative, an (oxodioxolenyl)methyl derivative, an N-Mannich base, imine or enamine. In addition, a secondary amine can be metabolically cleaved to generate a bioactive primary amine, or a tertiary amine can metabolically cleaved to generate a bioactive primary or secondary amine. For examples, see Simplicio, et al., Molecules 2008, 13, 519 and references therein.

The disclosure additionally includes use of clathrates of the compounds described herein, pharmaceutical compositions comprising the clathrates, and methods of use of the clathrates. In some embodiments, the disclosure is directed to clathrates of a disclosed compound of e.g., Formula Ia, Ib, Ic or Id, II, or III, or a pharmaceutical composition thereof.

As discussed above, the disclosure includes administration of pharmaceutical compositions comprising a pharmaceutically acceptable carrier or excipient and a compound described herein. A disclosed compound, or a pharmaceutically acceptable salt, solvate, clathrate or prodrug thereof, can be administered in pharmaceutical compositions comprising a pharmaceutically acceptable carrier or excipient. The excipient can be chosen based on the expected route of administration of the composition in therapeutic applications. The route of administration of the composition depends on the condition to be treated. For example, intravenous injection may be suitable for treatment of a systemic disorder and oral administration may be suitable to treat a gastrointestinal disorder. The route of administration and the dosage of the composition to be administered can be determined by the skilled artisan without undue experimentation in conjunction with standard dose-response studies. Relevant circumstances to be considered in making those determinations include the condition or conditions to be treated, the choice of composition to be administered, the age, weight, and response of the individual patient, and the severity of the patient's symptoms. A pharmaceutical composition comprising a disclosed compound or a pharmaceutically acceptable salt, solvate, clathrate or prodrug, can be administered by a variety of routes including, but not limited to, parenteral, oral, pulmonary, ophthalmic, nasal, rectal, vaginal, aural, topical, buccal, transdermal, intravenous, intramuscular, subcutaneous, intradermal, intraocular, intracerebral, intralymphatic, intraarticular, intrathecal and intraperitoneal. The compositions can also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the pharmacologic agent or composition. Examples of such diluents are distilled water, physiological phosphate-buffered saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like. Pharmaceutical compositions can also include large, slowly metabolized macromolecules such as proteins, polysaccharides such as chitosan, polylactic acids, polyglycolic acids and copolymers (such as latex functionalized SEPHAROSE^{™}, agarose, cellulose, and the like), polymeric amino acids, amino acid copolymers, and lipid aggregates (such as oil droplets or liposomes).

The compositions can be administered parenterally such as, for example, by intravenous, intramuscular, intrathecal or subcutaneous injection. Parenteral administration can be accomplished by incorporating a composition into a solution or suspension. Such solutions or suspensions may also include sterile diluents such as water for injection, saline solution, fixed oils, polyethylene glycols, glycerine, propylene glycol or other synthetic solvents. Parenteral formulations may also include antibacterial agents such as, for example, benzyl alcohol or methyl parabens, antioxidants such as, for example, ascorbic acid or sodium bisulfite and chelating agents such as EDTA. Buffers such as acetates, citrates or phosphates and agents for the adjustment of tonicity such as sodium chloride or dextrose may also be added. The parenteral preparation can be enclosed in ampules, disposable syringes or multiple dose vials made of glass or plastic.

Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are suitable liquid carriers, particularly for injectable solutions.

Injectable formulations can be prepared either as liquid solutions or suspensions; solid forms suitable for solution in, or suspension in, liquid vehicles prior to injection can also be prepared. The preparation also can also be emulsified or encapsulated in liposomes or micro particles such as polylactide, polyglycolide, or copolymer for enhanced adjuvant effect, as discussed above [Langer, Science 249: 1527, 1990 and Hanes, Advanced Drug Delivery Reviews 28: 97-119, 1997]. The compositions and pharmacologic agents described herein can be administered in the form of a depot injection or implant preparation which can be formulated in such a manner as to permit a sustained or pulsatile release of the active ingredient.

Additional formulations suitable for other modes of administration include oral, intranasal, and pulmonary formulations, suppositories, transdermal applications and ocular delivery. For suppositories, binders and carriers include, for example, polyalkylene glycols or triglycerides; such suppositories can be formed from mixtures containing the active ingredient in the range of about 0.5% to about 10%, or about 1% to about 2%. Oral formulations include excipients, such as pharmaceutical grades of mannitol, lactose, starch, magnesium stearate, sodium saccharine, cellulose, and magnesium carbonate. Topical application can result in transdermal or intradermal delivery. Transdermal delivery can be achieved using a skin patch or using transferosomes. [Paul et al., Eur. J. Immunol. 25: 3521-24, 1995; Cevc et al., Biochem. Biophys. Acta 1368: 201-15, 1998].

For the purpose of oral therapeutic administration, the pharmaceutical compositions can be incorporated with excipients and used in the form of tablets, troches, capsules, elixirs, suspensions, syrups, wafers, chewing gums and the like. Tablets, pills, capsules, troches and the like may also contain binders, excipients, disintegrating agent, lubricants, glidants, sweetening agents, and flavoring agents. Some examples of binders include microcrystalline cellulose, gum tragacanth or gelatin. Examples of excipients include starch or lactose. Some examples of disintegrating agents include alginic acid, corn starch and the like. Examples of lubricants include magnesium stearate or potassium stearate. An example of a glidant is colloidal silicon dioxide. Some examples of sweetening agents include sucrose, saccharin and the like. Examples of flavoring agents include peppermint, methyl salicylate, orange flavoring and the like. Materials used in preparing these various compositions should be pharmaceutically pure and non-toxic in the amounts used. In another embodiment, the composition is administered as a tablet or a capsule.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor, and the like. For vaginal administration, a pharmaceutical composition may be presented as pessaries, tampons, creams, gels, pastes, foams or spray.

The pharmaceutical composition can also be administered by nasal administration. As used herein, nasally administering or nasal administration includes administering the composition to the mucus membranes of the nasal passage or nasal cavity of the patient. As used herein, pharmaceutical compositions for nasal administration of a composition include therapeutically effective amounts of the compounds prepared by well-known methods to be administered, for example, as a nasal spray, nasal drop, suspension, gel, ointment, cream or powder. Administration of the composition may also take place using a nasal tampon or nasal sponge.

For topical administration, suitable formulations may include biocompatible oil, wax, gel, powder, polymer, or other liquid or solid carriers. Such formulations may be administered by applying directly to affected tissues, for example, a liquid formulation to treat infection of conjunctival tissue can be administered dropwise to the subject's eye, or a cream formulation can be administered to the skin.

Rectal administration includes administering the pharmaceutical compositions into the rectum or large intestine. This can be accomplished using suppositories or enemas. Suppository formulations can easily be made by methods known in the art. For example, suppository formulations can be prepared by heating glycerin to about 120 °C, dissolving the pharmaceutical composition in the glycerin, mixing the heated glycerin after which purified water may be added, and pouring the hot mixture into a suppository mold.

Transdermal administration includes percutaneous absorption of the composition through the skin. Transdermal formulations include patches, ointments, creams, gels, salves and the like.

In addition to the usual meaning of administering the formulations described herein to any part, tissue or organ whose primary function is gas exchange with the external environment, for purposes of the present disclosure, "pulmonary" will also mean to include a tissue or cavity that is contingent to the respiratory tract, in particular, the sinuses. For pulmonary administration, an aerosol formulation containing the active agent, a manual pump spray, nebulizer or pressurized metered-dose inhaler as well as dry powder formulations are contemplated. Suitable formulations of this type can also include other agents, such as antistatic agents, to maintain the disclosed compounds as effective aerosols.

A drug delivery device for delivering aerosols comprises a suitable aerosol canister with a metering valve containing a pharmaceutical aerosol formulation as described and an actuator housing adapted to hold the canister and allow for drug delivery. The canister in the drug delivery device has a head space representing greater than about 15% of the total volume of the canister. Often, the compound intended for pulmonary administration is dissolved, suspended or emulsified in a mixture of a solvent, surfactant and propellant. The mixture is maintained under pressure in a canister that has been sealed with a metering valve.

The disclosure also encompasses the treatment of a condition associated with a dysfunction in proteostasis in a subject comprising administering to said subject an effective amount of a first compound of Formula Ia, Ib, Ic or Id, a second compound of Formula II, and a third compound of Formula III or IV, that enhances, improves or restores proteostasis of a protein. Proteostasis refers to protein homeostasis. Dysfunction in protein homeostasis is a result of protein misfolding, protein aggregation, defective protein trafficking or protein degradation. For example, the disclosure encompasses administering a first compound of Formula Ia, Ib, Ic or Id, a second compound of Formula II, and a third compound of Formula III or IV, that corrects protein misfolding, reduces protein aggregation, corrects or restores protein trafficking and/or affects protein degradation for the treatment of a condition associated with a dysfunction in proteostasis. In some aspects of the disclosure, a first compound of Formula Ia, Ib, Ic or Id, a second compound of Formula II, and a third compound of Formula III or IV, that corrects protein misfolding and/or corrects or restores protein trafficking is administered. In cystic fibrosis, the mutated or defective enzyme is the cystic fibrosis transmembrane conductance regulator (CFTR). One of the most common mutations of this protein is ΔF508 which is a deletion (Δ) of three nucleotides resulting in a loss of the amino acid phenylalanine (F) at the 508th (508) position on the protein. As described above, mutated cystic fibrosis transmembrane conductance regulator exists in a misfolded state and is characterized by altered trafficking as compared to the wild type CFTR. Additional exemplary proteins of which there can be a dysfunction in proteostasis, for example that can exist in a misfolded state, include, but are not limited to, glucocerebrosidase, hexosamine A, aspartylglucosaminidase, α-galactosidase A, cysteine transporter, acid ceramidase, acid α-L-fucosidase, protective protein, cathepsin A, acid β-glucosidase, acid β-galactosidase, iduronate 2-sulfatase, α-L-iduronidase, galactocerebrosidase, acid α -mannosidase, acid β -mannosidase, arylsulfatase B, arylsulfatase A, *N*-acetylgalactosamine-6-sulfate sulfatase, acid β -galactosidase, *N-*acetylglucosamine-1-phosphotransferase, acid sphingmyelinase, NPC-1, acid α-glucosidase, β-hexosamine B, heparin *N*-sulfatase, α -*N*-acetylglucosaminidase, α -glucosaminide *N-*acetyltransferase, *N*-acetylglucosamine-6-sulfate sulfatase, α -*N*-acetylgalactosaminidase, α - neuramidase, β -glucuronidase, β-hexosamine A and acid lipase, polyglutamine, α - synuclein, TDP-43, superoxide dismutase (SOD), Aβ peptide, tau protein transthyretin and insulin. The disclosed compounds may be used to restore proteostasis (e.g., correct folding and/or alter trafficking) of the proteins described above.

Protein conformational diseases encompass gain of function disorders and loss of function disorders. In one embodiment, the protein conformational disease is a gain of function disorder. The terms "gain of function disorder," "gain of function disease," "gain of toxic function disorder" and "gain of toxic function disease" are used interchangeably herein. A gain of function disorder is a disease characterized by increased aggregation-associated proteotoxicity. In these diseases, aggregation exceeds clearance inside and/or outside of the cell. Gain of function diseases include, but are not limited to, neurodegenerative diseases associated with aggregation of polyglutamine, Lewy body diseases, amyotrophic lateral sclerosis, transthyretin-associated aggregation diseases, Alzheimer's disease, Machado-Joseph disease, cerebral B-amyloid angiopathy, retinal ganglion cell degeneration, tautopathies (progressive supranuclear palsy, corticobasal degeneration, frontotemporal lobar degeneration), cerebral hemorrhage with amyloidosis, Alexander disease, Serpinopathies, familial amyloidotic neuropathy, senile systemic amyloidosis, ApoAI amyloidosis, ApoAII amyloidosis, ApoAIV amyloidosis, familial amyloidosis of the Finnish type, lysozyme amyloidosis, fibrinogen amyloidosis, dialysis amyloidosis, inclusion body myositis/myopathy, cataracts, medullary thyroid carcinoma, cardiac atrial amyloidosis, pituitary prolactinoma, hereditary lattice corneal dystrophy, cutaneous lichen amyloidosis, corneal lactoferrin amyloidosis, corneal lactoferrin amyloidosis, pulmonary alveolar proteinosis, odontogenic tumor amyloid, seminal vesical amyloid, sickle cell disease, critical illness myopathy, von Hippel-Lindau disease, spinocerebellar ataxia 1, Angelman syndrome, giant axon neuropathy, inclusion body myopathy with Paget disease of bone, frontotemporal dementia (IBMPFD) and prion diseases. Neurodegenerative diseases associated with aggregation of polyglutamine include, but are not limited to, Huntington's disease, dentatorubral and pallidoluysian atrophy, several forms of spino-cerebellar ataxia, and spinal and bulbar muscular atrophy. Alzheimer's disease is characterized by the formation of two types of aggregates: extracellular aggregates of Aβ peptide and intracellular aggregates of the microtubule associated protein tau. Transthyretin-associated aggregation diseases include, for example, senile systemic amyloidoses and familial amyloidotic neuropathy. Lewy body diseases are characterized by an aggregation of α-synuclein protein and include, for example, Parkinson's disease, Lewy body dementia (LBD) and multiple system atrophy (SMA). Prion diseases (also known as transmissible spongiform encephalopathies or TSEs) are characterized by aggregation of prion proteins. Exemplary human prion diseases are Creutzfeldt-Jakob Disease (CJD), Variant Creutzfeldt-Jakob Disease, Gerstmann-Straussler-Scheinker Syndrome, Fatal Familial Insomnia and Kuru. In another embodiment, the misfolded protein is alpha-1 anti-trypsin.

In a further embodiment, the protein conformation disease is a loss of function disorder. The terms "loss of function disease" and "loss of function disorder" are used interchangeably herein. Loss of function diseases are a group of diseases characterized by inefficient folding of a protein resulting in excessive degradation of the protein. Loss of function diseases include, for example, lysosomal storage diseases. Lysosomal storage diseases are a group of diseases characterized by a specific lysosomal enzyme deficiency which may occur in a variety of tissues, resulting in the build-up of molecules normally degraded by the deficient enzyme. The lysosomal enzyme deficiency can be in a lysosomal hydrolase or a protein involved in the lysosomal trafficking. Lysosomal storage diseases include, but are not limited to, aspartylglucosaminuria, Fabry's disease, Batten disease, Cystinosis, Farber, Fucosidosis, Galactasidosialidosis, Gaucher's disease (including Types 1, 2 and 3), Gm1 gangliosidosis, Hunter's disease, Hurler-Scheie's disease, Krabbe's disease, α-Mannosidosis, β-Mannosidosis, Maroteaux-Lamy's disease, Metachromatic Leukodystrophy, Morquio A syndrome, Morquio B syndrome, Mucolipidosis II, Mucolipidosis III, Neimann-Pick Disease (including Types A, B and C), Pompe's disease, Sandhoff disease, Sanfilippo syndrome (including Types A, B, C and D), Schindler disease, Schindler-Kanzaki disease, Sialidosis, Sly syndrome, Tay-Sach's disease and Wolman disease.

In another embodiment, the disease associated with a dysfunction in proteostasis is a cardiovascular disease. Cardiovascular diseases include, but are not limited to, coronary artery disease, myocardial infarction, stroke, restenosis and arteriosclerosis. Conditions associated with a dysfunction of proteostasis also include ischemic conditions, such as, ischemia/reperfusion injury, myocardial ischemia, stable angina, unstable angina, stroke, ischemic heart disease and cerebral ischemia.

In yet another embodiment, the disease associated with a dysfunction in proteostasis is diabetes and/or complications of diabetes, including, but not limited to, diabetic retinopathy, cardiomyopathy, neuropathy, nephropathy, and impaired wound healing.

In a further embodiment, the disease associated with a dysfunction in proteostasis is an ocular disease including, but not limited to, age-related macular degeneration (AMD), diabetic macular edema (DME), diabetic retinopathy, glaucoma, cataracts, retinitis pigmentosa (RP) and dry macular degeneration.

In yet additional embodiments, the method of the disclosure is directed to treating a disease associated with a dysfunction in proteostasis, wherein the disease affects the respiratory system or the pancreas. In certain additional embodiments, the methods of the disclosure encompass treating a condition selected from the group consisting of polyendocrinopathy/hyperinsulinemia, diabetes mellitus, Charcot-Marie Tooth syndrome, Pelizaeus-Merzbacher disease, and Gorham's Syndrome.

Additional conditions associated with a dysfunction of proteostasis include hemoglobinopathies, inflammatory diseases, intermediate filament diseases, drug-induced lung damage and hearing loss. The disclosure also encompasses methods for the treatment of hemoglobinopathies (such as sickle cell anemia), an inflammatory disease (such as inflammatory bowel disease, colitis, ankylosing spondylitis), intermediate filament diseases (such as non-alcoholic and alcoholic fatty liver disease) and drug induced lung damage (such as methotrexate-induced lung damage). The disclosure additionally encompasses methods for treating hearing loss, such as noise-induced hearing loss, aminoglycoside-induced hearing loss, and cisplatin-induced hearing loss.

Additional conditions include those associated with a defect in protein trafficking and that can be treated according to methods of the disclosure include: PGP mutations, hERG trafficking mutations, nephrongenic diabetes insipidus mutations in the arginine-vasopressin receptor 2, persistent hyperinsulinemic hypoglycemia of infancy (PHH1) mutations in the sulfonylurea receptor 1, and α1AT.

The disclosure is illustrated by the following examples which are not meant to be limiting in any way.

### EXEMPLIFICATION

The compounds described herein can be prepared in a number of ways based on the teachings contained herein and synthetic procedures known in the art. In the description of the synthetic methods described below, it is to be understood that all proposed reaction conditions, including choice of solvent, reaction atmosphere, reaction temperature, duration of the experiment and workup procedures, can be chosen to be the conditions standard for that reaction, unless otherwise indicated. It is understood by one skilled in the art of organic synthesis that the functionality present on various portions of the molecule should be compatible with the reagents and reactions proposed. Substituents not compatible with the reaction conditions will be apparent to one skilled in the art, and alternate methods are therefore indicated. The starting materials for the examples are either commercially available or are readily prepared by standard methods from known materials. At least some of the compounds identified as "intermediates" herein are contemplated as compounds of the disclosure.

### Example 1: N-trans-3-(5-((R)-1-hydroxyethyl)-1,3,4-oxadiazol-2-yl)cyclobutyl)-3-phenylisoxazole-5-carboxamide (Compound 1)

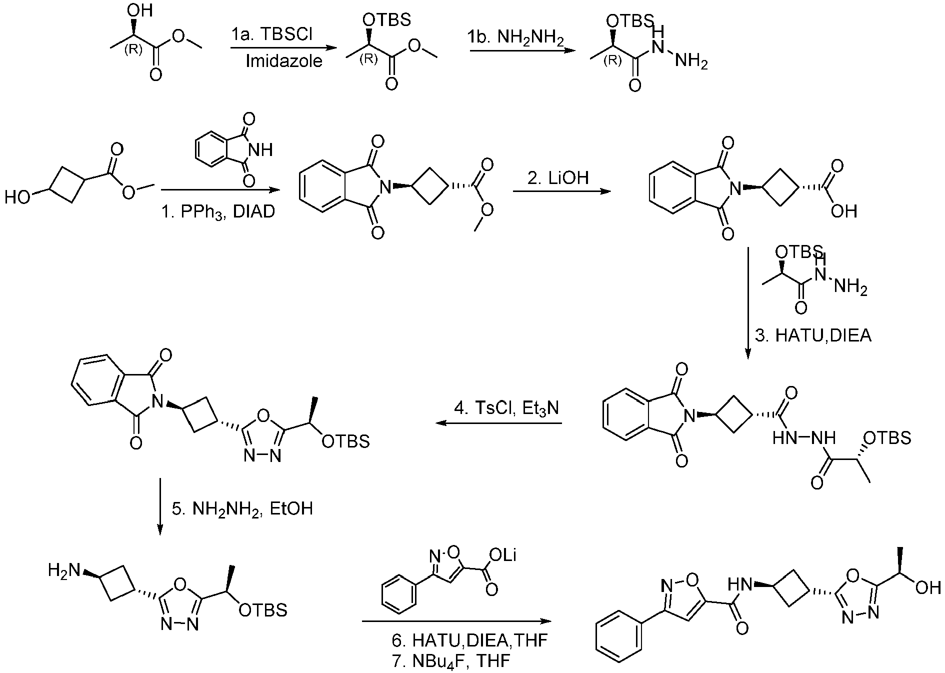

**Step 1a: methyl (2*R*)-2-[(tert-butyldimethylsilyl)oxy]propanoate:** into a 250-mL round-bottom flask was placed a solution of methyl (2*R*)-2-hydroxypropanoate (5 g, 48.03 mmol, 1.00 eq.) and imidazole (6.5 g, 95.59 mmol, 2.00 eq.) in dichloromethane (100 mL), followed by the dropwise addition of a solution of tert-butyl(chloro)dimethylsilane (8.7 g, 57.72 mmol, 1.20 eq.) in dichloromethane (50 mL) at 0 °C. The resulting solution was stirred for 2 h at room temperature. The reaction was quenched by the addition of 100 mL of water/ice. The resulting solution was extracted with dichloromethane (3 x 100 mL) and the organic layers combined. The resulting mixture was washed with brine (3 x 50 mL), dried over anhydrous sodium sulfate and concentrated under vacuum to afford 7 g (67%) of methyl (2*R*)-2-[(tert-butyldimethylsilyl)oxy]propanoate as a colorless oil.

**Step 1b: (2*R*)-2-[(tert-butyldimethylsilyl)oxy]propanehydrazide:** into a 250-mL round-bottom flask was placed a solution of methyl (2*R*)-2-[(tertbutyldimethylsilyl)oxy]propanoate (7 g, 32.06 mmol, 1.00 eq.) in ethanol (100 mL). To the solution was added hydrazine (10 g, 159.81 mmol, 5.00 eq., 80%). The resulting solution was stirred for 15 h at 90 °C in an oil bath. The resulting solution was quenched by the addition of water/ice. The resulting solution was extracted with ethyl acetate (3 x 100 mL) and the organic layers combined. The resulting mixture was washed with brine (2 x 100 mL), dried over anhydrous sodium sulfate and concentrated under vacuum to afford 6.5 g (93%) of (2*R*)-2-[(tert-butyldimethylsilyl)oxy]propanehydrazide as a colorless oil. LC-MS (ES, *m*/*z*): [M+1]⁺ = 219.

**Step 1: methyl (*trans*-3-(1,3-dioxo-2,3-dihydro-1H-isoindol-2-yl)cyclobutane-1-carboxylate:** into a 250-mL round-bottom flask, under nitrogen was placed a solution of methyl 3-cis-hydroxycyclobutane-1-carboxylate (8 g, 61.47 mmol, 1.00 eq.), 2,3-dihydro-1H-isoindole-1,3-dione (18.1 g, 123.02 mmol, 2.00 eq.) and triphenylphosphine (32.3 g, 123.15 mmol, 2.00 eq.) in THF (100 mL), followed by addition of DIAL) (24.9 g, 123.14 mmol, 2.00 eq.) dropwise with stirring at 0 °C. The resulting solution was stirred for 2.5 hours at room temperature. The resulting mixture was concentrated under vacuum. The residue was applied onto a silica gel column with ethyl acetate/petroleum ether (1:5). The crude product was re-crystallized from petroleum ether/ethyl acetate in the ratio of 10:1 to afford 7.2 g (45%) of methyl *trans*-3-(1,3-dioxo-2,3-dihydro-1H-isoindol-2-yl)cyclobutane-1-carboxylate as a white solid. LC-MS (ES, *m*/*z*): [M+1]⁺ = 260. ¹H-NMR (400MHz, CDCl₃): δ 7.85-7.82 (m, 2H), 7.74-7.71 (m, 2H), 5.08-5.04 (m, 1H), 3.75 (s, 3H), 3.34-3.32 (m, 1H), 3.20-3.12 (m, 2H), 2.66-2.60 (m, 2H).

**Step 2: *trans*-3-(1,3-dioxo-2,3-dihydro-1H-isoindol-2-yl)cyclobutane-1-carboxylic acid:** into a 100-mL round-bottom flask, was placed a solution of methyl *trans*-3-(1,3-dioxo-2,3-dihydro-1H-isoindol-2-yl)cyclobutane-1-carboxylate (7.2 g, 27.77 mmol, 1.00 eq.) in 1,4-dioxane (100 mL). To the solution was added 5M hydrogen chloride aqueous (10 mL). The resulting solution was stirred for 4 hours at 80 °C in an oil bath. The resulting mixture was concentrated under vacuum to afford 6.2 g (91%) of *trans*-3-(1,3-dioxo-2,3-dihydro-1H-isoindol-2-yl)cyclobutane-1-carboxylic acid as a white solid. LC-MS (ES, *m*/*z*): [M-1]⁻ = 244.

**Step 3: (2*R*)-2-[(tert-butyldimethylsilyl)oxy]-*N*-[*trans*-3-(1,3-dioxo-2,3-dihydro-1H-isoindol-2-yl)cyclobutyl]carbonyl]propanehydrazide:** into a 250-mL round-bottom flask, was placed a solution of *trans*-3-(1,3-dioxo-2,3-dihydro-1H-isoindol-2-yl)cyclobutane-1-carboxylic acid (6.2 g, 25.28 mmol, 1.00 eq.), (2*R*)-2-[(tertbutyldimethylsilyl)oxy]propanehydrazide (6.61 g, 30.27 mmol, 1.20 eq.) and HATU (14.4 g, 37.89 mmol, 1.50 eq.) in THF (100 mL), followed by the addition of DIEA (9.81 g, 75.91 mmol, 3.00 eq.) dropwise with stirring at 0 °C. The resulting solution was stirred for 1 hour at room temperature. The reaction was then quenched by the addition of 100 mL of water/ice. The resulting solution was extracted with ethyl acetate (3 x 50 mL) and the organic layers combined. The resulting mixture was washed with brine (2 x 50 mL), dried over anhydrous sodium sulfate and concentrated under vacuum. The residue was applied onto a silica gel column with ethyl acetate/petroleum ether (1:4) to afford 7 g (62%) of (2R)-2-[(tert-butyldimethylsilyl)oxy]-*N*-[ *trans*-3-(1,3-dioxo-2,3-dihydro-1H-isoindol-2-yl)cyclobutyl]carbonyl]*propanehydrazide* as colorless oil. LC-MS (ES, *m*/*z*): [M+1]⁺ = 446.

**Step 4: 2-[*tran*s-3-[5-[(1*R*)-1-[(tert-butyldimethylsilyl)oxy]ethyl]-1,3,4-oxadiazol-2-yl]cyclobutyl]-2,3-dihydro-1H-isoindole-1,3-dione:** into a 250-mL round-bottom flask was placed a solution of (2R)-2-[(tert-butyldimethylsilyl)oxy]-*N*-[[ *trans*-3-(1,3-dioxo-2,3-dihydro-1H-isoindol-2-yl)cyclobutyl]carbonyl]propanehydrazide (6.95 g, 15.60 mmol, 1.00 eq.) and TEA (7.89 g, 77.97 mmol, 5.00 eq.) in dichloromethane (100 mL), followed by addition of a solution of 4-methylbenzene-1-sulfonyl chloride (8.92 g, 46.79 mmol, 3.00 eq.) in dichloromethane (50 mL) dropwise with stirring at 0 °C. The resulting solution was stirred for 15 hours at room temperature. The reaction was then quenched by the addition of 100 mL of water/ice. The resulting solution was extracted with dichloromethane (2 x 50 mL) and the organic layers combined. The resulting mixture was washed with brine (2 x 50 mL), dried over anhydrous sodium sulfate and concentrated under vacuum. The crude product was purified by Flash-Prep-HPLC with the following conditions (IntelFlash-1): Column, C18; mobile phase, H₂O/CH₃CN = 100: 1 increasing to H₂O/CH₃CN = 1:100 within 30 min; Detector, UV 254 nm to afford 3.28 g (49%) of 2-[ *trans*-3-[5-[(1*R*)-1-[(tert-butyldimethylsilyl)oxy]ethyl]-1,3,4-oxadiazol-2-yl]cyclobutyl]-2,3-dihydro-1*H-*isoindole-1,3-dione as colorless oil. LC-MS (ES, *m*/*z*): [M+1]⁺ = 428. ¹H-NMR (400MHz, CDCl₃): δ 7.72-7.70 (m, 2H), 7.60-7.58 (m, 2H), 5.04-4.96 (m, 2H), 3.83-3.78 (m, 1H), 3.26-3.24 (m, 2H), 2.67-2.62 (m, 2H), 1.49-1.48 (d, *J* = 6.8Hz, 3H), 0.76 (s, 9H), 0.01 (s, 3H), 0.00 (s, 3H).

**Step 5: *trans*-3-[5-[(1*R*)-1-[(tert-butyldimethylsilyl)oxy]ethyl]-1,3,4-oxadiazol-2-yl]cyclobutan-1-amine:** into a 250-mL round-bottom flask, was placed a solution of 2-[ *trans*-3-[5-[(1*R*)-1-[(tert-butyldimethylsilyl)oxy]ethyl]-1,3,4-oxadiazol-2-yl]cyclobutyl]-2,3-dihydro-1H-isoindole-1,3-dione (1.18 g, 2.76 mmol, 1.00 eq.) in ethanol (100 mL). To the solution was added hydrazine hydrate (3.45 g, 55.13 mmol, 20.00 eq., 80%). The resulting solution was stirred for 3 hours at room temperature. The solids were filtered. The resulting mixture was concentrated under vacuum to afford 760 mg (crude) of *trans*-3-[5-[(1*R*)-1-[(tert-butyldimethylsilyl)oxy]ethyl]-1,3,4-oxadiazol-2-yl]cyclobutan-1-amine as colorless oil. LC-MS (ES, *m*/*z*): [M+1]⁺ = 298.

**Step 6: *N*-(trans-3-[5-[(1*R*)-1-[(tert-butyldimethylsilyl)oxy]ethyl]-1,3,4-oxadiazol-2-yl]cyclobutyl)-3-phenylisoxazole-5-carboxamide:** into a 100-mL round-bottom flask, was placed a solution of lithio 3-phenylisoxazole-5-carboxylate (300 mg, 1.54 mmol, 1.20 eq.), 3-[5-[(1*R*)-1-[(tert-butyldimethylsilyl)oxy]ethyl]-1,3,4-oxadiazol-2-yl]cyclobutan-1-amine (380 mg, 1.28 mmol, 1.00 eq.) and HATU (728 mg, 1.92 mmol, 1.50 eq.) in THF (50 mL). This was followed by the addition of DIEA (500 mg, 3.87 mmol, 3.00 eq.) dropwise with stirring at 0 °C. The resulting solution was stirred for 1 hour at room temperature. The resulting solution was diluted with 50 mL of water/ice. The resulting solution was extracted with ethyl acetate (3 x 50 mL) and the organic layers combined. The resulting mixture was washed with brine (2 x 30 mL), dried over anhydrous sodium sulfate and concentrated under vacuum to afford 300 mg (50%) of *N*-(*trans*-3-[5-[(1*R*)-1-[(tertbutyldimethylsilyl)oxy]ethyl]-1,3,4-oxadiazol-2-yl]cyclobutyl)-3-phenylisoxazole-5-carboxamide as an off-white crude solid. LC-MS (ES, *m*/*z*): [M+1]⁺ = 469.

**Step 7: *N*-(trans-3-[5-[(1*R*)-1-hydroxyethyl]-1,3,4-oxadiazol-2-yl]cyclobutyl)-3-phenylisoxazole-5-carboxamide:** into a 50-mL round-bottom flask, was placed a solution of *N*-(3-[*trans*-S-[(1*R*)-1-[(tert-butyldimethylsilyl)oxy]ethyl]-1,3,4-oxadiazol-2-yl]cyclobutyl)-3-phenylisoxazole-5-carboxamide (300 mg, 0.64 mmol, 1.00 eq.) and TBAF (1mol/L in tetrahydrofuran, 1 mL) in THF (5 mL). The resulting solution was stirred for 3 hours at room temperature and diluted with 20 mL of water. The resulting solution was extracted with ethyl acetate (3 x 30 mL) and the organic layers combined. The resulting mixture was washed with brine (2 x 10 mL), dried over anhydrous sodium sulfate and concentrated under vacuum. The residue was applied onto a silica gel column with dichloromethane/methanol (20:1). The crude product was purified by Flash-Prep-HPLC with the following conditions (IntelFlash-1): Column, C18; mobile phase, H₂O/CH₃CN = 100:1 increasing to H₂O/CH₃CN = 1:100 within 30 min; Detector, UV 254 nm to afford 149.2 mg (66%) of *N*-(*trans*-3-[5-[(1*R*)-1-hydroxyethyl]-1,3,4-oxadiazol-2-yl]cyclobutyl)-3-phenylisoxazole-5-carboxamide (Compound A) as a white solid. LC-MS (ES, *m*/*z*): [M+1]⁺ = 355; ¹H NMR (400MHz, DMSO-*d*₆): δ 9.48-9.46 (d, *J* = 7.6 Hz, 1H), 7.96-7.93 (m, 2H), 7.67 (s, 1H), 7.56-7.54 (m, 3H), 5.95-5.94 (d, *J* = 5.6Hz, 1H), 4.95-4.89 (m, 1H), 4.73-4.63 (m, 1H), 3.77-3.71 (m, 1H), 2.73-2.50 (m, 4H), 1.50-1.48 (d, *J* = 6.8Hz, 3H).

### Example 2: Preparation of Sodium 5-((1R)-1-(tetrahydro-2H-pyran-4-yl)ethoxy)-8-methyl-2-(3-methyl-1-benzofuran-2-yl)quinoline-4-carboxylate (Compound 2)

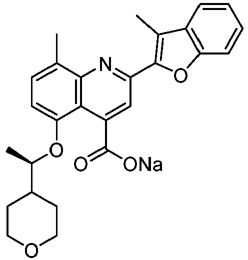

A. 4-(1-(4-Methyl-3-nitrophenoxy)ethyl)tetrahydro-2H-pyran. To a 1000-mL 3-necked round-bottom flask purged and maintained with an inert atmosphere of nitrogen was placed a solution of 1-(tetrahydro-2H-pyran-4-yl)ethan-1-ol (10 g, 76.8 mmol) in THF (400 mL) then 4-methyl-3-nitrophenol (10.6 g, 69.2 mmol) and PPh₃ (30.2 g, 115.1 mmol) were added. This was followed by the addition of DIAD (23.3 g, 115.2 mmol) at rt. The reaction was stirred for 5 h at rt and concentrated under reduced pressure. The residue was treated with water and extracted with DCM. The organic extracts were combined, dried over anhydrous Na₂SO₄, and concentrated under reduced pressure. The residue was purified by column chromatography eluting with EtOAc/petroleum ether (1:50) affording 10 g (49%) of the title compound as a yellow oil.
B. 2-Methyl-5-(1-(tetrahydro-2H-pyran-4-yl)ethoxy)aniline. To a 500-mL round-bottom flask purged and maintained with an inert atmosphere of N₂ was placed a solution of 4-(1-(4-Methyl-3-nitrophenoxy)ethyl)tetrahydro-2H-pyran (3.00 g, 11.3 mmol, as prepared in the previous step) in MeOH (200 mL) then Raney Ni (300 mg) was added. The solution was degassed and back filled with hydrogen and stirred for 2 h at rt. The H₂ was purged then the solids were removed by filtration. The filtrate was concentrated under reduced pressure affording 2.70 g of the title compound as a yellow oil. Mass Spectrum (LCMS, ESI pos): Calcd. for C₁₄H₂₂NO₂⁺: 236.2 (M+H); Found: 236.2.
C. 5-((1R)-1-(tetrahydro-2H-pyran-4-yl)ethoxy)-8-methyl-2-(3-methyl-1-benzofuran-2-yl)quinoline-4-carboxylic acid and 5-((1S)-1-(tetrahydro-2H-pyran-4-yl)ethoxy)-8-methyl-2-(3-methyl-1-benzofuran-2-yl)quinoline-4-carboxylic acid. To a 20-mL sealed tube was placed a solution of 2-methyl-5-(1-(tetrahydro-2H-pyran-4-yl)ethoxy)aniline (1.00 g, 4.26 mmol, as prepared in the previous step) in EtOH (10 mL) then 3-methyl-1-benzofuran-2-carbaldehyde (680 mg, 4.26 mmol) and 2-oxopropanoic acid (749 mg, 8.52 mmol) were added. The reaction was stirred overnight at 110°C then the reaction was cooled to rt and the solids were collected by filtration. The isomers were separated by Prep-SFC (Column, EnantioPak-A1, 250mm*50mm,5um; mobile phase, CO₂(50%), MeOH Preparative(50%); Detector, UV 254nm) affording 210 mg (11%) of 5-((1R)-1-(tetrahydro-2H-pyran-4-yl)ethoxy)-8-methyl-2-(3-methyl-1-benzofuran-2-yl)quinoline-4-carboxylic acid as a yellow solid and 190 mg (10%) of 5-((1S)-1-(tetrahydro-2H-pyran-4-yl)ethoxy)-8-methyl-2-(3-methyl-1-benzofuran-2-yl)quinoline-4-carboxylic acid as a yellow solid.
D. Sodium 5-((1R)-1-(Tetrahydro-2H-pyran-4-yl)ethoxy)-8-methyl-2-(3-methyl-1-benzofuran-2-yl)quinoline-4-carboxylate. To a 50-mL round-bottom flask purged and maintained with an inert atmosphere of nitrogen was placed a solution of 5-((1R)-1-(tetrahydro-2H-pyran-4-yl)ethoxy)-8-methyl-2-(3-methyl-1-benzofuran-2-yl)quinoline-4-carboxylic acid (210 mg, 0.45 mmol) in MeOH (1 mL) then 0.05NNaOH (9.0 mL, 0.45 mmol) was added. The reaction was stirred for 3 h at rt then the solvent was removed under reduced pressure affording 219.6 mg (99%) of the title compound as a light yellow solid. Mass Spectrum (LCMS, ESI pos): Calcd. for C₂₇H₂₈NO₅⁺: 446.2 (M+H); Found: 446.2. ¹H NMR (300 MHz, DMSO-*d₆*): δ 7.74 (d, *J* = 7.2 Hz, 1H), 7.66 (d, *J* = 8.1 Hz, 1H), 7.56 (s, 1H), 7.45-7.30 (m, 3H), 6.80 (d, *J* = 8.1 Hz, 1H), 4.36-4.34 (m, 1H), 3.91-3.84 (m, 2H), 3.30-3.26 (m, 2H), 2.84 (s, 3H), 2.64 (s, 3H), 1.99-1.92 (m, 2H), 1.79-1.74 (m, 1H), 1.39-1.31 (m, 2H), 1.20 (d, *J* = 6.0 Hz, 3H). HPLC purity (254 nm): 99.3%.

### Example 3: Preparation of N-(5-Hydroxy-2,4-bis(trimethylsilyl)phenyl)-4-oxo-1,4-dihydroquinoline-3-carboxamide (Compound 3)

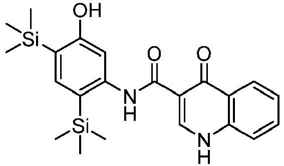

A. N-(2,4-Dibromo-5-hydroxyphenyl)acetamide. To a 3000-mL round-bottom flask was placed a solution of N-(3-hydroxyphenyl)acetamide (15 g, 99.23 mmol) in MeOH (300 mL) and DCM (1.2 L) then Py·Br₃ (70.18 g, 220.13 mmol) was added in portions at rt over 1 h. The reaction was stirred overnight at rt then concentrated under reduced pressure. The resulting mixture was diluted with 800 mL of water and extracted with EtOAc (2x1.5 L). The organic extracts were combined, dried over anhydrous Na₂SO₄, and concentrated under reduced pressure. The residue was purified by column chromatography eluting with EtOAc/petroleum ether (1:1) affording 9 g of the title compound as a white solid. Mass Spectrum (LCMS, ESI pos): Calcd. for C₈H₈Br₂NO₂⁺: 307.9 (M+H); Found: 308.1.
B. 5-Amino-2,4-dibromophenol. To a 500-mL round-bottom flask was placed a solution of N-(2,4-dibromo-5-hydroxyphenyl)acetamide (10.4 g, 33.66 mmol, as prepared in the previous step) in H₂O (130 mL) then conc. HCl (46 mL) was added. The reaction was stirred for 3 h at 100°C then NaOAc was added to adjust the pH to 7. The solids were removed by filtration then the filtrate was extracted with EtOAc (3x300 mL). The organic extracts were combined, dried over anhydrous Na₂SO₄, and concentrated under reduced pressure affording 8.2 g of the title compound as a yellow solid. Mass Spectrum (LCMS, ESI pos): Calcd. for C₆H₆Br₂NO⁺: 265.9 (M+H); Found: 266.2.
C. N-(2,4-Dibromo-5-hydroxyphenyl)-4-oxo-1,4-dihydroquinoline-3-carboxamide. To a 100-mL round-bottom flask was placed a solution of 4-oxo-1,4-dihydroquinoline-3-carboxylic acid (945 mg, 5.00 mmol) in DMF (25 mL) then 5-amino-2,4-dibromophenol (1.99 g, 7.46 mmol, as prepared in the previous step), HATU (3.8 g, 9.99 mmol), and DIEA (2 g, 15.48 mmol) were added. The reaction was stirred for 2 days at 85°C then diluted with 100 mL of water and extracted with EtOAc (3x200 mL). The organic extracts were combined and concentrated under reduced pressure. The crude product was triturated with EtOAc affording 1.1 g of the title compound as a white solid. Mass Spectrum (LCMS, ESI pos): Calcd. for C₁₆H₁₁Br₂N₂O₃⁺: 436.9 (M+H); Found: 437.1.
D. N-[5-Hydroxy-2,4-bis(trimethylsilyl)phenyl]-4-oxo-1,4-dihydroquinoline-3-carboxamide. To a 20-mL sealed tube purged and maintained with an inert atmosphere of nitrogen, was placed a solution of N-(2,4-dibromo-5-hydroxyphenyl)-4-oxo-1,4-dihydroquinoline-3-carboxamide (200 mg, 0.46 mmol, as prepared in the previous step) in DMPU (3 mL) then hexamethyldisilane (533 mg, 3.64 mmol), Pd₂(dba)₃·CHCl₃ (24 mg, 0.02 mmol), JohnPhos (20 mg, 0.07 mmol), and KF/Al₂O₃ (50%, 265 mg, 4.57 mmol) were added. The reaction was stirred for 16 h at 110°C, cooled to rt, and filtered. The filtrate was diluted with 50 mL of water and extracted with EtOAc (3x50 mL). The organic extracts were combined, dried over anhydrous Na₂SO₄, and concentrated under reduced pressure. The crude product was purified by Prep-HPLC (HPLC-10: Column, X Bridge C18 OBD Prep Column, 10 µm, 19 mm X 250 mm; mobile phase, Water (10 mmol/L NH₄HCO₃) and MeCN (60.0% MeCN up to 85.0% in 8 min); Detector, UV 254/220nm) affording 8.2 mg of the title compound as a white solid. Mass Spectrum (LCMS, ESI pos): Calcd. for C₂₂H₂₉N₂O₃Si₂⁺: 425.2 (M+H); Found: 425.1. ¹H NMR (400 MHz, DMSO-*d*₆): δ 12.89 (br s, 1H), 11.86 (s, 1H), 9.60 (s, 1H), 8.87 (s, 1H), 8.32 (d, *J* = 8.4 Hz, 1H), 7.90-7.70 (m, 2H), 7.50 (t, *J* = 7.6 Hz, 1H), 7.35 (d, *J* = 7.6 Hz, 2H), 0.31 (s, 9H), 0.25 (s, 9H). HPLC purity (254 nm): 96.1%.

### Example 4: Preparation of Sodium 8-Methyl-2-(3-methylbenzofuran-2-yl)-5-((2,2,6,6-tetramethyltetrahydro-2H-pyran-4-yl)methoxy)quinoline-4-carboxylate (Compound 4) - Provided for reference

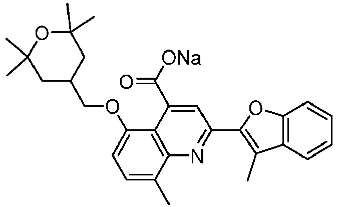

A. 4-(Methoxymethylene)-2,2,6,6-tetramethyltetrahydro-2H-pyran. To a 250-mL 3-necked round-bottom flask was placed a solution of methoxymethyl)triphenylphosphonium chloride (16.4 g, 53.54 mmol) in THF (150 mL) then LiHMDS (48 mL of 1.1M THF solution) was added dropwise with stirring and the resulting solution was stirred for 1h at - 20°C. A solution of 2,2,6,6-tetramethyltetrahydro-4H-pyran-4-one (4 g, 25.60 mmol) in THF (30 mL) was then added dropwise with stirring at -20°C, then warmed to rt and stirred for 12 h. The reaction was quenched by the addition of water and was extracted with DCM. The organic extracts were combined and concentrated under reduced pressure. The residue was purified by column chromatography eluting with EtOAc/petroleum ether (1:80) affording 3.7 g of the title compound as a yellow oil.
B. 2,2,6,6-Tetramethyltetrahydro-2H-pyran-4-carbaldehyde. To a 100-mL round-bottom flask, was placed a solution of 4-(methoxymethylene)-2,2,6,6-tetramethyltetrahydro-2H-pyran (2.5 g, 13.57 mmol, as prepared in the previous step) in H_{2O}:THF (1:1) (20 mL) then toluenesulfonic acid (3.2 g, 18.58 mmol) was added. The reaction was stirred for 2 h at rt then quenched by the addition of water. The mixture was extracted with EtOAc, then the organic extratcs were combined and concentrated under reduced pressure affording 2 g of the title compound as a yellow oil.
C. (2,2,6,6-Tetramethyltetrahydro-2H-pyran-4-yl)methanol. To a 100-mL round-bottom flask was placed a solution of 2,2,6,6-tetramethyltetrahydro-2H-pyran-4-carbaldehyde (1.1 g, 6.46 mmol, as prepared in the previous step) in MeOH (10 mL) then the solution was cooled to 0°C and NaBH₄ (123 mg, 3.34 mmol) was added in small portions with stirring. The reaction was stirred at 0°C for 30 min then quenched by the addition of water. The resulting mixture was extracted with EtOAc and the combined organic layers were concentrated under reduced pressure affording 1 g of the title compound as a yellow oil.
D. 2,2,6,6-Tetramethyl-4-((4-methyl-3-nitrophenoxy)methyl)tetrahydro-2H-pyran. To a 100-mL 3-necked round-bottom flask was placed a solution of (2,2,6,6-tetramethyltetrahydro-2H-pyran-4-yl)methanol (1 g, 5.81 mmol, as prepared in the previous step) in THF (20 mL) then 4-methyl-3-nitrophenol (889 mg, 5.81 mmol) and PPh₃ (1.98 g, 7.55 mmol) were added. DIAD (1.53 g, 7.57 mmol) was added dropwise to the reaction mixture and stirred at rt for 12 h. The reaction was concentrated under reduced pressure then the residue was purified by column chromatography eluting with EtOAc/petroleum ether (1:80) affording 1.2 g of the title compound as a yellow oil.
E. 2-Methyl-5-((2,2,6,6-tetramethyltetrahydro-2H-pyran-4-yl)methoxy)aniline. To a 100-mL round-bottom flask was placed a solution of 2,2,6,6-tetramethyl-4-((4-methyl-3-nitrophenoxy)methyl)tetrahydro-2H-pyran (1 g, 3.25 mmol, as prepared in the previous step) in MeOH (10 mL) then Raney Ni (100 mg) was added. The solution was degassed and back-filled with hydrogen and stirred at rt for 1 h. The hydrogen was vented and the solids were removed by filtration. The filtrate was concentrated under reduced pressure affording 800 mg of the title compound as a yellow oil. Mass Spectrum (LCMS, ESI pos): Calcd. for C₁₇H₂₈NO₂⁺: 278.2 (M+H); Found: 278.2.
F. 8-Methyl-2-(3-methylbenzofuran-2-yl)-5-((2,2,6,6-tetramethyltetrahydro-2H-pyran-4-yl)methoxy)quinoline-4-carboxylic acid. To a 10-mL sealed tube was placed a solution of 2-methyl-5-((2,2,6,6-tetramethyltetrahydro-2H-pyran-4-yl)methoxy)aniline (350 mg, 1.26 mmol, as prepared in the previous step) in EtOH (3 mL) then 3-methyl-1-benzofuran-2-carbaldehyde (202 mg, 1.26 mmol) and 2-oxopropanoic acid (222 mg, 2.52 mmol) were added. The reaction was stirred at 120°C for 12 h then cooled to rt. The solids were removed by filtration affording 70 mg of the title compound as a yellow solid. Mass Spectrum (LCMS, ESI pos): Calcd. for C₃₀H₃₄NO₅⁺: 488.2 (M+H); Found: 488.2.
G. Sodium 8-Methyl-2-(3-methylbenzofuran-2-yl)-5-((2,2,6,6-tetramethyltetrahydro-2H-pyran-4-yl)methoxy)quinoline-4-carboxylate. To a 50-mL round-bottom flask was placed a solution of 8-methyl-2-(3-methylbenzofuran-2-yl)-5-((2,2,6,6-tetramethyltetrahydro-2H-pyran-4-yl)methoxy)quinoline-4-carboxylic acid (60 mg, 0.12 mmol, as prepared in the previous step) in MeOH (5 mL) then NaOH in water (0.01N) (12mL of a 0.01N solution) was added. The reaction was stirred at rt for 20 min the solution was lyophilized affording 54.9 mg of the title compound as a yellow solid. Mass Spectrum (LCMS, ESI pos): Calcd. for C₃₀H₃₄NO₅⁺: 488.2 (M+H-Na); Found: 488.2. ¹H NMR (400 MHz, DMSO-*d*₆): δ 7.72 (d, *J* = 7.2 Hz, 1H), 7.65 (d, *J* = 8.0 Hz, 1H), 7.55 (s, 1H), 7.44-7.33 (m, 2H), 7.31 (t, *J* = 7.2 Hz, 1H), 6.73 (d, *J* = 8.0 Hz, 1H), 3.79 (d, *J* = 6.8 Hz, 2H), 2.83 (s, 3H), 2.64 (s, 3H), 2.45-2.36 (m, 1H), 1.98-1.94 (m, 2H), 1.25 (s, 6H), 1.11 (s, 6H), 1.04-0.98 (m, 2H). HPLC purity (254 nm): 98.5%.

### Example 5: Preparation of N-(5-Hydroxy-2-(trifluoromethyl)-4-(trimethylsilyl)phenyl)-4-oxo-1,4-dihydroquinoline-3-carboxamide (Compound 5) - Provided for reference

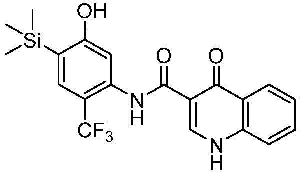

A. 2-Bromo-4-(trifluoromethyl)phenyl methyl carbonate. To a 250-mL round-bottom flask was placed a solution of 2-bromo-4-(trifluoromethyl)phenol (4.8 g, 19.92 mmol) in DCM (100 mL) then TEA (4 g, 39.53 mmol) and DMAP (250 mg, 2.05 mmol) were added, followed by the dropwise addition of methyl chloroformate (3.8 g, 40.21 mmol) with stirring at 0°C. The reaction was stirred at rt for 3 h then quenched by the addition of water (100 mL) and extracted with DCM (3x100 mL). The organic extracts were combined, dried over anhydrous Na₂SO₄, and concentrated under reduced pressure. The residue was purified by column chromatography eluting with EtOAc/petroleum ether (0-5%) affording 4.0 g of the title compound as yellow oil.
B. 2-Bromo-5-nitro-4-(trifluoromethyl)phenyl methyl carbonate. To a 250-mL round-bottom flask was placed a solution of 2-bromo-4-(trifluoromethyl)phenyl methyl carbonate (4 g, 13.38 mmol, as prepared in the previous step) in H₂SO₄ (20 mL) then HNO₃/H₂SO₄ (1:1, 10 mL) was added dropwise with stirring at 0°C. The reaction was stirred at rt for 3 h then quenched by the addition of 300 mL of water/ice and extracted with DCM (3x100 mL). The organic extracts were combined, dried over anhydrous Na₂SO₄, and concentrated under reduced pressure. The residue was purified by column chromatography eluting with EtOAc/petroleum ether (0-50%) affording 1.0 g of the title compound as a light yellow solid.
C. 5-Nitro-4-(trifluoromethyl)-2-(trimethylsilyl)phenol. To a 40-mL sealed tube purged and maintained with an inert atmosphere of nitrogen, was placed a solution of 2-bromo-5-nitro-4-(trifluoromethyl)phenyl methyl carbonate (300 mg, 0.87 mmol, as prepared in the previous step) in DMPU (3 mL) then hexamethyldisilane (2.6 g, 17.76 mmol), PdCh (154 mg, 0.87 mmol), and K₃PO₄ (369 mg, 1.74 mmol) were added. The reaction was stirred at 110°C for 24 h, then cooled and filtered. The filtrate was diluted with water (50 mL) and extracted with EtOAc (3x100 mL). The organic extracts were combined, dried over anhydrous Na₂SO₄, and concentrated under reduced pressure. The residue was purified by column chromatography eluting with EtOAc/petroleum ether (0-50%) affording 160 mg of the title compound as light yellow oil. Mass Spectrum (LCMS, ESI neg): Calcd. for C₁₀H₁₁F₃NO₃Si⁻: 278.0 (M+H); Found: 278.0.
D. 5-Amino-4-(trifluoromethyl)-2-(trimethylsilyl)phenol. To a 25-mL round-bottom flask was placed a solution of 5-nitro-4-(trifluoromethyl)-2-(trimethylsilyl)phenol (160 mg, 0.57 mmol, as prepared in the previous step) and Ni(OAc)₂ (102 mg, 0.58 mmol) in methanol/THF (2 mL), then NaBH₄ (22 mg, 0.58 mmol) was added in portions at 0°C. The reaction was stirred at 0°C for 10 min, then filtered, diluted with water (100 mL), and extracted with EtOAc (3x100 mL). The organic extracts were combined, dried over anhydrous Na₂SO₄, and concentrated under reduced pressure affording 120 mg of the title compound as a yellow oil. Mass Spectrum (LCMS, ESI pos): Calcd. for C₁₀H₁₅F₃NOSi⁺: 250.1 (M+H); Found: 250.1.
E. N-(5-Hydroxy-2-(trifluoromethyl)-4-(trimethylsilyl)phenyl)-4-oxo-1,4-dihydroquinoline-3-carboxamide. To a 100-mL round-bottom flask was placed a solution of 5-amino-4-(trifluoromethyl)-2-(trimethylsilyl)phenol (120 mg, 0.48 mmol, as prepared in the previous step) in DMF (10 mL), then 4-oxo-1,4-dihydroquinoline-3-carboxylic acid (137 mg, 0.72 mmol), HATU (365 mg, 0.96 mmol), and DIEA (186 mg, 1.44 mmol) were added. The reaction was stirred at rt for 16 h, diluted with water (100 mL), and extracted with EtOAc (3x100 mL). The organic extracts were combined, dried over anhydrous Na₂SO₄, and concentrated under reduced pressure. The residue was purified by Chiral-Prep-HPLC (Column, Chiralpak IA, 2*25cm, 5um; mobile phase, Hex- and ethanol- (hold 10.0% ethanol-in 13 min); Detector, UV 220/254nm) affording in 6.6 mg of the title compound as a white solid. Mass Spectrum (LCMS, ESI pos): Calcd. for C₂₀H₂₀F₃N₂O₃Si⁺: 421.1 (M+H); Found: 421.2. ¹HNMR (300 MHz, DMSO-*d*₆): δ 13.15 (br s, 1H), 12.67 (s, 1H), 10.37 (s, 1H), 8.86 (s, 1H), 8.31 (d, *J* = 8.1 Hz, 1H), 7.99 (s, 1H), 7.84-7.73 (m, 2H), 7.54-7.49 (m, 1H), 7.45 (s, 1H) , 0.25 (s, 9H). HPLC purity (254 nm): 99.8%.

### Example 6: Preparation of N-[4-tert-Butyl-5-hydroxy-2-(trimethylsilyl)phenyl]-4-oxo-1,4-dihydroquinoline-3-carboxamide (Compound 6) - Provided for reference

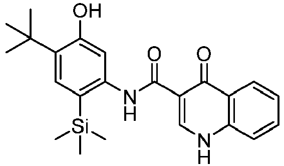

A. 4-Bromo-2-tert-butylphenyl methyl carbonate. To a 100-mL 3-necked round-bottom flask purged and maintained with an inert atmosphere of nitrogen, was placed a solution of 4-bromo-2-tert-butylphenol (1 g, 4.36 mmol), TEA (887 mg, 8.77 mmol), and DMAP (54 mg, 0.42 mmol) in DCM (10 mL) then the solution was cooled to 0°C and methyl chloroformate (494 mg, 5.23 mmol) was added. The reaction was stirred for 3 h at 0°C then quenched by the addition of 20 mL of water and extracted with DCM (3x20 mL). The organic extracts were combined and concentrated under reduced pressure affording 1.5 g of the title compound as a yellow oil.
B. 4-Bromo-2-tert-butyl-5-nitrophenyl methyl carbonate. To a 50-mL round-bottom flask was placed a solution of 4-bromo-2-tert-butylphenyl methyl carbonate (1.2 g, 4.18 mmol, as prepared in the previous step) in conc. sulfuric acid (5 mL) then the solution was cooled to 0°C and KNO₃ (0.55 g) was added in portions. The reaction was stirred for 2 h at rt then quenched by the addition of 30 mL of water/ice and extracted with DCM (3x30 mL). The organic extracts were combined and concentrated under reduced pressure. The residue was purified by column chromatography eluting with EtOAc/petroleum ether (1:20) affording 1.1 g of the title compound as a yellow solid. ¹H NMR (300 MHz, CD₃OD): δ 7.87 (s, 1H), 7.77 (s, 1H), 3.91 (s, 3H), 1.35 (s, 9H).
C. 5-Amino-4-bromo-2-tert-butylphenyl methyl carbonate. To a 25-mL round-bottom flask was placed a solution of 4-bromo-2-tert-butyl-5-nitrophenyl methyl carbonate (664 mg, 2.00 mmol, as prepared in the previous step) in THF (10 mL) and AcOH (2 mL) then Fe powder (1000 mg, 17.91 mmol) was added in portions over 15 min at 66°C. The reaction was stirred for 8 h at 66°C, cooled to rt, and filtered. The filtrate was concentrated under reduced pressure. The residue was purified by column chromatography eluting with EtOAc/petroleum ether (1:1) affording 450 mg of the title compound as a yellow solid.
D. 4-Bromo-2-tert-butyl-5-(4-oxo-1,4-dihydroquinoline-3-amido)phenyl methyl carbonate. To a 25-mL round-bottom flask was placed a solution of 5-amino-4-bromo-2-tert-butylphenyl methyl carbonate (604 mg, 2.00 mmol, as prepared in the previous step) in DMF (10 mL) then 4-oxo-1,4-dihydroquinoline-3-carboxylic acid (567 mg, 3.00 mmol), DIEA (516 mg, 3.99 mmol), and HATU (1524 mg, 4.00 mmol) were added. The reaction was stirred for 16 h at 65°C then quenched by the addition of 50 mL of water/ice and extracted with EtOAc (3x25 mL). The organic extracts were combined and concentrated under reduced pressure. The residue was purified by column chromatography eluting with EtOAc/petroleum ether (1:1) affording 800 mg of the title compound as a yellow solid. Mass Spectrum (LCMS, ESI pos): Calcd. for C₂₂H₂₂BrN₂O₅⁺: 473.1 (M+H); Found: 473.1.
E. N-[4-tert-Butyl-5-hydroxy-2-(trimethylsilyl)phenyl]-4-oxo-1,4-dihydroquinoline-3-carboxamide. To a 40-mL sealed tube was placed a solution of 4-bromo-2-tert-butyl-5-(4-oxo-1,4-dihydroquinoline-3-amido)phenyl methyl carbonate (47.3 mg, 0.10 mmol, as prepared in the previous step) in DMPU (1 mL) then [Rh(cod)₂]BF₄ (20.3 mg, 0.05 mmol), hexamethyldisilane (1 mL), and K₃PO₄ (84.9 mg, 0.40 mmol) were added under nitrogen. The reaction was stirred for 16 h at 110°C then quenched by the addition of 10 mL of ice/water and extracted with EtOAc (3x10 mL). The organic extracts were combined, dried over Na₂SO₄, and concentrated under reduced pressure. The crude product was purified by Prep-HPLC (HPLC-10: Column, X Bridge Shield RP18 OBD Column, 19*250mm, 10um; mobile phase, Water (10 mmol/L NH₄HCO₃) and MeCN (60.0% MeCN up to 90.0% in 11 min); Detector, UV 254/220 nm) affording 10.1 mg of the title compound as a white solid. Mass Spectrum (LCMS, ESI pos): Calcd. for C₂₃H₂₉N₂O₃Si⁺: 409.2 (M+H); Found: 409.2. ¹H NMR (300 MHz, DMSO-*d₆*): δ 11.73 (s, 1H), 8.96 (s, 1H), 9.51 (s, 1H), 8.43 (s, 1H), 8.30 (d, *J=* 8.4 Hz, 1H), 7.71-7.81 (m, 2H), 7.49 (t, *J* = 8.1 Hz, 1H), 7.29 (s, 1H), 7.21 (s, 1H), 1.34 (s, 9H), 0.28 (s, 9H). HPLC purity (254 nm): 98.1%.

### Example 7:

General procedures for the preparation of diclosed compounds are outlined in Scheme I and Scheme II. The disclosed compounds may be prepared, for example, either by base-mediated condensation of an aromatic aldehyde with a suitably functionalized isatin derivative (Scheme I), or three-component coupling between an aromatic aldehyde, a functionalized aniline, and an alpha-keto acid as shown in Scheme II. Further functional group conversion provides a sulfonamide.

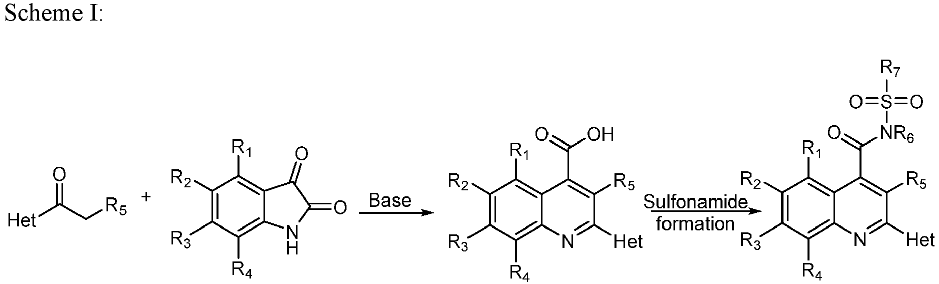

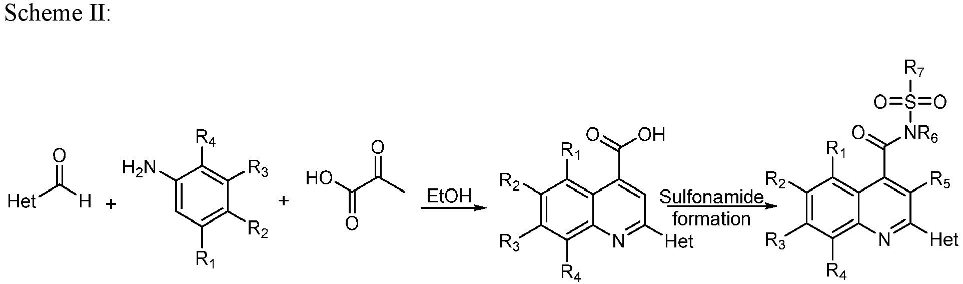

For example, R₁, R₂, R₃, R₄, R₅, R₆, R₇, and Het may be groups readily contemplated by a person of skill in the art. For example, R₇ may be, e.g, C₁₋₆alkyl, C₃₋₆cycloalkyl, phenyl, heteroaryl (e.g., pyridinyl, pyrrazolyl or thiazolyl) or heterocyclyl (e.g., morpholinyl or thiazolyl). For example, R₆ may be, e.g, hydrogen or C₁₋₆alkyl. For example, R₁ may be C₁₋₆alkoxy, wherein C₁₋₆alkoxy may optionally be substituted by phenyl or a 5-6 membered monocyclic heteroaryl (e.g., tetrahydropyranyl, optionally substituted by one, two, three, or four substituents each independently selected from hydroxyl, C₁₋₆alkyl, C₁₋₆alkoxy, and oxo). For example, R₂, R₃, R₄, and R₅ may be independently selected from hydrogen or C₁₋₆alkyl. For example, Het may be, e.g., benzofuranyl or benothiofuranyl.

### Example 8: CFTR activity assays

### i. Ussing measurements

Ussing measurements were used to measure CFTR activity. In this method, primary lung epithelial cells (hBEs) with a cystic fibrosis causing mutation were differentiated for a minimum of 4 weeks in an air-liquid interface on SnapWell^{™} filter plates prior to the Ussing measurements. Cells were apically mucus-washed for 30 minutes prior to treatment with compounds. The basolateral media was removed and replaced with media containing the compounds of interest diluted to its final concentration from DMSO or aqueous stocks. The cells were treated with the potentiator, continuously with the corrector and the amplifier, for 24 hours prior to assessment of CFTR activity in the Ussing chamber assay. The treated cells were incubated at 37 °C and 5% CO₂ for 24 hours. At the end of the treatment period, the cells on filters were transferred to the Ussing chamber and equilibrated for 30 minutes. The short-circuit current was measured in voltage clamp-mode (V_{hold} = 0 mV), and the entire assay was conducted at a temperature of 36°C -36.5°C. Once the voltages stabilized, the chambers were clamped, and data were recorded by pulse readings every 5 seconds. Following baseline current stabilization, the following additions were applied and the changes in current and resistance of the cells were monitored:
1. Benzamil to the apical chamber to inhibit ENaC sodium channel.
2. Forskolin to both chambers to activate ΔF508-CFTR by phosphorylation.
3. CFTRinh-172 to the apical chamber to inhibit ΔF508-CFTR Cl- conductance.

The forskolin-sensitive current and inhibitable current (that potentiated current that was blocked by CFTRinh-172) were measured as the specific activity of the ΔF508-CFTR channel, and increase in response to compounds in this activity over that observed in vehicle-treated samples were identified as the correction of ΔF508-CFTR function imparted by the compounds tested. The results are shown below in Table 1.

**Table 1. Percent activity in F508/F508del and G542X/F508del HBEs (relative to wild type)**

| | F508del/F508del | G542X/F508del |
|---|---|---|
| Compound 1 (10 µM) + Compound 2 (10 µM) + Compound 3 (1 µM) | 105 ± 6 % | 63 ± 2 % |
| DMSO | 9 ± 1 % | 4 ± 1 % |

In a separate study, chronic double combination treatment of CFTR homozygous F508del HBE cells with Compound 2 and Compound 3 gave an average maximum measured effect ("MME") of 19.2 µA/cm² (CFTRinh172-inhibitable current). These results corresponded to an average of 58.68% of non-CF HBE activity (designated as 32.72 µA/cm²). The results of three experiments and their combined average is shown below in Table 2.

**Table 2.**

| Experiment Number | EC₅₀ (nM) | EC₉₀ (nM) | MME (µA/cm²) |
|---|---|---|---|
| 1 | 11.7 | 236 | 17.6 |
| 2 | 3.80 | 55.2 | 19.4 |
| 3 | 6.63 | 45.4 | 21.2 |
| Average | 6.08 | 83.7 | 19.2 |

### Example 9

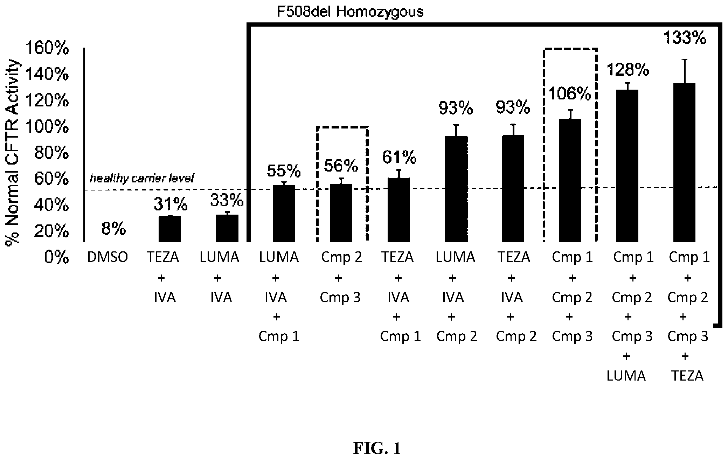

A study was conducted to determine the *in vitro* CFTR modulating effects of doublet and triplet combinations of disclosed CFTR modulators in CFTR homozygous F508del patient cells. The results are shown in FIG. 1. TEZA = tezacaftor (((R)-1-(2,2-difluorobenzo[d][1,3]dioxol-5-yl)-N (1-(2,3-dihydroxypropyl)-6-fluoro-2-(1-hydroxy-2-methylpropan-2-yl)-1H-indol-5-yl)cyclopropane-1-carboxamide), 3 µM); IVA = ivacaftor (*N-*(2,4-Di-tert-butyl-5-hydroxyphenyl)-4-oxo-1,4-dihydroquinoline-3-carboxamide; 1 µM); LUMA = lumacaftor ((3-(6-(1-(2,2-difluorobenzo[d][1,3]dioxol-5-yl)cyclopropane carboxamido)-3-methylpyridin-2-yl)benzoic acid, 3 µM); Cmp 1 = Compound 1 (N-trans-3-(5-((*R*)-1-hydroxyethyl)-1,3,4-oxadiazol-2-yl)cyclobutyl)-3-phenylisoxazole-5-carboxamide, 10 µM); Cmp 2 = Compound 2 (sodium 5-((1*R*)-1-(tetrahydro-2H-pyran-4-yl)ethoxy)-8-methyl-2-(3-methyl-1-benzofuran-2-yl)quinoline-4-carboxylate, 10 µM); Cmp 3 = Compound 3 (*N-*(5-hydroxy-2,4-bis(trimethylsilyl)phenyl)-4-oxo-1,4-dihydroquinoline-3-carboxamide, 1 µM).

### Example 10

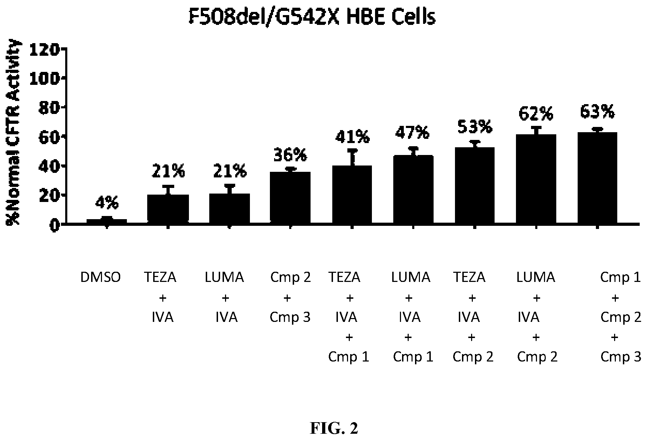

A study was conducted to determine the *in vitro* CFTR modulating effects of doublet and triplet combinations of disclosed CFTR modulators in CFTR heterozygous F508del/G542X patient cells. The results are shown in FIG. 2. TEZA = tezacaftor (((*R*)-1-(2,2-difluorobenzo[d][1,3]dioxol-5-yl)-N (1-(2,3-dihydroxypropyl)-6-fluoro-2-(1-hydroxy-2-methylpropan-2-yl)-1H-indol-5-yl)cyclopropane-1-carboxamide), 3 µM); IVA = ivacaftor (*N-*(2,4-Di-tert-butyl-5-hydroxyphenyl)-4-oxo-1,4-dihydroquinoline-3-carboxamide; 1 µM); LUMA = lumacaftor ((3-(6-(1-(2,2-difluorobenzo[d][1,3]dioxol-5-yl)cyclopropane carboxamido)-3-methylpyridin-2-yl)benzoic acid, 3 µM); Cmp 1 = Compound 1 (*N*-trans-3-(5-((*R*)-1-hydroxyethyl)-1,3,4-oxadiazol-2-yl)cyclobutyl)-3-phenylisoxazole-5-carboxamide, 10 µM); Cmp 2 = Compound 2 (sodium 5-((1*R*)-1-(tetrahydro-2H-pyran-4-yl)ethoxy)-8-methyl-2-(3-methyl-1-benzofuran-2-yl)quinoline-4-carboxylate, 10 µM); Cmp 3 = Compound 3 (*N-*(5-hydroxy-2,4-bis(trimethylsilyl)phenyl)-4-oxo-1,4-dihydroquinoline-3-carboxamide, 1 µM).

## Claims

1. A compound represented by 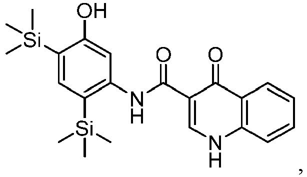
or a pharmaceutically acceptable salt thereof,
for use in a method of treating a disease associated with decreased cystic fibrosis transmembrane conductance regulator (CFTR) activity in a patient in need thereof, the method comprising administering said compound in combination with compounds: 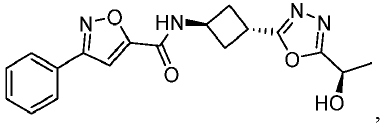 or a pharmaceutically acceptable salt thereof, and 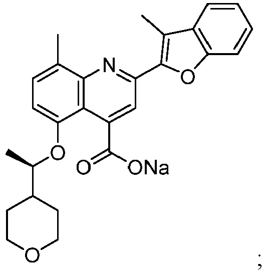
wherein the disease is selected from the group consisting of cystic fibrosis, congenital bilateral absence of vas deferens (CBAVD), acute, recurrent, or chronic pancreatitis, disseminated bronchiectasis, asthma, allergic pulmonary aspergillosis, chronic obstructive pulmonary disease (COPD), chronic sinusitis, dry eye disease, protein C deficiency, A-β-lipoproteinemia, lysosomal storage disease, type 1 chylomicronemia, mild pulmonary disease, lipid processing deficiencies, type 1 hereditary angioedema, coagulation-fibrinolyis, hereditary hemochromatosis, CFTR-related metabolic syndrome, chronic bronchitis, constipation, pancreatic insufficiency, hereditary emphysema, Sjogren's syndrome, familial hypercholesterolemia, I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDGtype 1, congenital hyperthyroidism, osteogenesis imperfecta, hereditary hypofibrinogenemia, ACT deficiency, Diabetes insipidus (DI), neurophyseal DI, nephrogenic DI, Charcot-Marie Tooth syndrome, Perlizaeus-Merzbacher disease, Alzheimer's disease, Parkinson's disease, amyotrophic lateral sclerosis, progressive supranuclear palsy, Pick's disease, Huntington's disease, spinocerebellar ataxia type I, spinal and bulbar muscular atrophy, dentatorubral pallidoluysian, myotonic dystrophy, hereditary Creutzfeldt-Jakob disease (due to prion protein processing defect), Fabry disease, cholestatic liver disease (primary biliary cirrhosis (PBC), primary sclerosing cholangitis (PSC)), and Straussler-Scheinker syndrome.

2. The compound for use of claim 1, wherein the patient has one or more CFTR mutations selected from the group consisting of ΔF508, S549N, G542X, G551D, R117H, N1303K, W1282X, R553X, 621+1G>T, 1717-1G>A, 3849+10kbC>T, 2789+5G>A, 3120+1G>A, I507del, R1162X, 1898+1G>A, 3659delC, G85E, D1152H, R560T, R347P, 2184insA, A455E, R334W, Q493X, E56K, P67L, R74W, D110E, D110H, R117C, G178R, E193K, L206W, R347H, R352Q, A455E, S549R, G551S, D579G, S945L, S997F, F1052V, K1060T, A1067T, G1069R, R1070Q, R1070W, F1074L, G1244E, S1251N, S1255P, D1270N, G1349D, and 2184delA CFTR.

3. The compound for use of any one of claims 1-2, wherein the patient has a ΔF508 and a G542X mutation.

4. The compound for use of any one of claims 1-3 wherein the patient has a homozygous ΔF508 mutation.

5. The compound for use of any one of claims 1-4, wherein the disease is cystic fibrosis.

6. The compound for use of any one of claims 1-5, wherein the patient is a human patient.

7. A CFTR amplifier compound for use in a method of treating cystic fibrosis in a patient homozygous for the ΔF508 mutation or having a ΔF508/G542X mutation, the method comprising administering to the patient an effective combination, sequentially or substantially simultaneously, of the CFTR amplifier compound, a CFTR corrector compound, and a CFTR potentiator compound, wherein:
the amplifier compound is represented by: 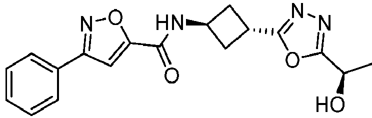 or a pharmaceutically acceptable salt thereof;
the corrector compound is represented by: 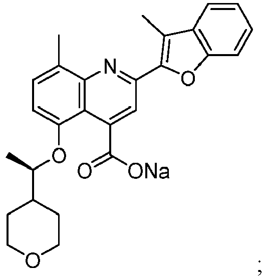 and
the potentiator compound is represented by: 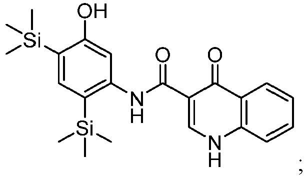
or a pharmaceutically acceptable salt thereof.

8. The compound for use of any one of claims 1-7, wherein the method further comprises administering an additional CFTR corrector.

9. The compound for use of claim 8, wherein the CFTR corrector is selected from the group consisting of (3-(6-(1-(2,2-difluorobenzo[d][1,3]dioxol-5-yl)cyclopropanecarboxamido)-3-methylpyridin-2-yl)benzoic acid (lumacaftor), deuterated lumacaftor, ((R)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropane-1-carboxamide (tezacaftor), deuterated tezacaftor, VX-152, VX-440, VX-445, VX-659, GLPG2222, GLPG2851, GLPG2737, GLPG3221 and VX-983.

## Patentansprüche

1. Verbindung, wiedergegeben durch 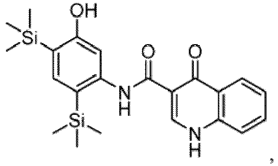
oder ein pharmazeutisch unbedenkliches Salz davon zur Verwendung bei einem Verfahren zur Behandlung einer mit verringerter Aktivität des CFTR (Cystic Fibrosis Transmembrane Conductance Regulator) bei einem Patienten, bei dem diesbezüglicher Bedarf besteht, wobei das Verfahren das Verabreichen der Verbindung in Kombination mit Verbindungen: 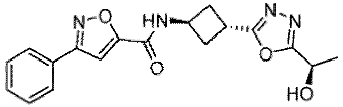 oder einem pharmazeutisch unbedenklichen Salz davon und 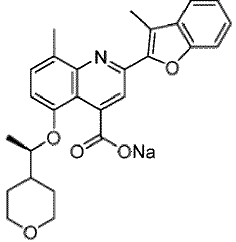
umfasst;
wobei die Erkrankung aus der Gruppe bestehend aus zystischer Fibrose, kongenitaler bilateraler Aplasie des Vas deferens (CBAVD), akuter, wiederkehrender oder chronischer Pankreatitis, disseminierter Bronchiektasie, Asthma, allergischer pulmonaler Aspergillose, chronisch-obstruktiver Lungenerkrankung (COPD), chronischer Sinusitis, Keratokonjunktivitis sicca, Protein-C-Defizienz, A-β-Lipoproteinämie, lysosomaler Speicherkrankheit, Chylomikronämie vom Typ 1, milder Lungenkrankheit, Defizienzen bei der Lipidverarbeitung, erblichem Angioödem vom Typ 1, Koagulation-Fibrinolyse, erblicher Hämochromatose, CFTR-bedingtem metabolischem Syndrom, chronischer Bronchitis, Verstopfung, Bauchspeicheldrüseninsuffizienz, erblichem Emphysem, Sjögren-Syndrom, familiärer Hypercholesterinämie, I-Zellkrankheit/Pseudo-Hurler, Mukopolysaccharidosen, Sandhof/Tay-Sachs, Crigler-Najjar Typ II, Polyendokrinopathie/Hyperinsulämie, Diabetes mellitus, Laron-Kleinwuchs, Myleoperoxidasedefizienz, primärem Hypoparathyroidismus, Melanom, Glycanose CDG Typ 1, angeborener Hyperthyreose, Osteogenesis imperfecta, erblicher Hypofibrinogenämie, ACT-Defizienz, Diabetes insipidus (DI), neurophysealem DI, nephrogenem DI, Morbus Charcot-Marie-Tooth, Perlizaeus-Merzbacher-Krankheit, Alzheimer-Krankheit, Parkinson-Krankheit, amyotropher Lateralsklerose, progressiver supranukleärer Blickparese, Pick-Krankheit, Chorea Huntington, spinozerebellärer Ataxie Typ I, spinobulbärer Muskelatrophie, dentatorubro-pallidoluysischer, myotoner Dystrophie, erblicher Creutzfeldt-Jakob-Krankheit (aufgrund eines Defekts bei der Prionproteinverarbeitung), Fabry-Krankheit, cholestatischer Leberkrankheit (primärer biliärer Zirrhose (PBC), primärer sklerosierender Cholangitis (PSC)) und Straussler-Scheinker-Syndrom ausgewählt ist.

2. Verbindung zur Verwendung nach Anspruch 1, wobei der Patient ein oder mehrere CFTR-Mutationen aufweist, die aus der Gruppe bestehend aus ΔF508, S549N, G542X, G551D, R117H, N1303K, W1282X, R553X, 621+1G>T, 1717-1G>A, 3849+10kbC>T, 2789+5G>A, 3120+1G>A, I507del, R1162X, 1898+1G>A, 3659delC, G85E, D1152H, R560T, R347P, 2184insA, A455E, R334W, Q493X, E56K, P67L, R74W, D110E, D110H, R117C, G178R, E193K, L206W, R347H, R352Q, A455E, S549R, G551S, D579G, S945L, S997F, F1052V, K1060T, A1067T, G1069R, R1070Q, R1070W, F1074L, G1244E, S1251N, S1255P, D1270N, G1349D und 2184delA CFTR ausgewählt sind.

3. Verbindung zur Verwendung nach einem der Ansprüche 1-2, wobei der Patient eine ΔF508- und eine G542X-Mutation aufweist.

4. Verbindung zur Verwendung nach einem der Ansprüche 1-3, wobei der Patient eine homozygote ΔF508-Mutation aufweist.

5. Verbindung zur Verwendung nach einem der Ansprüche 1-4, wobei es sich bei der Erkrankung um zystische Fibrose handelt.

6. Verbindung zur Verwendung nach einem der Ansprüche 1-5, wobei es sich bei dem Patienten um einen menschlichen Patienten handelt.

7. CFTR-Verstärkerverbindung zur Verwendung bei einem Verfahren zur Behandlung von zystischer Fibrose bei einem Patienten, der für die ΔF508-Mutation homozygot ist oder eine ΔF508/G542X-Mutation aufweist, wobei das Verfahren das aufeinanderfolgende oder weitgehend gleichzeitige Verabreichen einer wirksamen Kombination der CFTR-Verstärkerverbindung, einer CFTR-Korrekturverbindung und einer CFTR-Potentiatorverbindung an den Patienten umfasst, wobei:
die Verstärkerverbindung wiedergegeben wird durch: 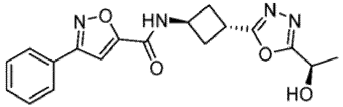 oder ein pharmazeutisch unbedenkliches Salz davon;
die Korrekturverbindung wiedergegeben wird durch: 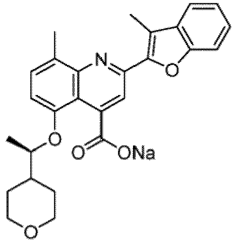 und
die Potentiatorverbindung wiedergegeben wird durch: 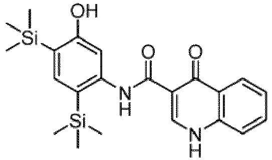
oder ein pharmazeutisch unbedenkliches Salz davon.

8. Verbindung zur Verwendung nach einem der Ansprüche 1-7, wobei das Verfahren ferner das Verabreichen eines zusätzlichen CFTR-Korrektors umfasst.

9. Verbindung zur Verwendung nach Anspruch 8, wobei der CFTR-Korrektor aus der Gruppe bestehend aus (3-(6-(1-(2,2-Difluorbenzo[d][1,3]dioxol-5-yl)cyclopropancarboxamido)-3-methylpyridin-2-yl)benzoesäure (Lumacaftor), deuteriertem Lumacaftor, ((R)-1-(2,2-Difluorbenzo[d][1,3]-dioxol-5-yl)-N-(1-(2,3-dihydroxypropyl)-6-fluor-2-(1-hydroxy-2-methylpropan-2-yl)-1H-indol-5-yl)cyclopropan-1-carboxamid (Tezacaftor), deuteriertem Tezacaftor, VX-152, VX-440, VX-445, VX-659, GLPG2222, GLPG2851, GLPG2737, GLPG3221 und VX-983 ausgewählt ist.

## Revendications

1. Composé représenté par 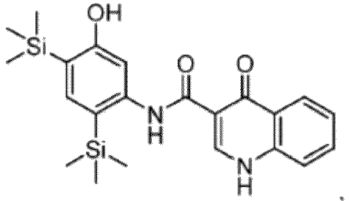
ou sel pharmaceutiquement acceptable correspondant,
pour une utilisation dans un procédé de traitement d'une maladie associée à l'activité du régulateur de la conductance transmembranaire de la fibrose kystique diminuée (CFTR) chez un patient qui en a besoin, le procédé comprenant une administration dudit composé en combinaison avec les composés : 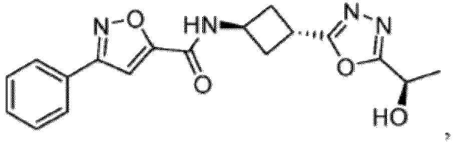 ou un sel pharmaceutiquement acceptable correspondant, et 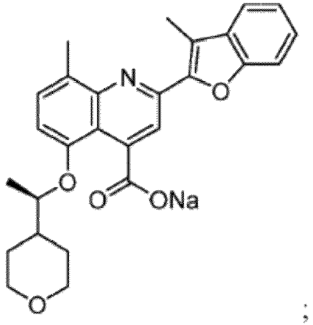
la maladie étant choisie dans le groupe constitué par la fibrose kystique, l'absence congénitale bilatérale des canaux déférents (ACBCD), la pancréatite aiguë, récurrente ou chronique, la bronchectasie disséminée, l'asthme, l'aspergillose pulmonaire allergique, la bronchopneumopathie chronique obstructive (BPCO), la sinusite chronique, la sécheresse oculaire, le déficit en protéine C, l'A-β-lipoprotéinémie, la maladie de surcharge lysosomale, la chylomicronémie de type 1, la maladie pulmonaire légère, les déficits du traitement des lipides, l'angio-oedème héréditaire de type 1, la coagulation-fibrinolyse, l'hémochromatose héréditaire, le syndrome métabolique lié au CFTR, la bronchite chronique, la constipation, l'insuffisance pancréatique, l'emphysème héréditaire, le syndrome de Sjögren, l'hypercholestérolémie familiale, la maladie des cellules I/pseudo-Hurler, les mucopolysaccharidoses, le syndrome de Sandhof/Tay-Sachs, le syndrome de Crigler-Najjar de type II, la polyendocrinopathie/hyperinsulémie, le diabète sucré, le nanisme de Laron, le déficit en myléoperoxydase, l'hypoparathyroïdie primaire, le mélanome, la glycanose CDG de type 1, l'hyperthyroïdie congénitale, l'ostéogenèse imparfaite, l'hypofibrinogénémie héréditaire, le déficit en ACT, le diabète insipide (DI), le DI neurophysaire, le DI néphrogénique, le syndrome de Charcot-Marie Tooth, la maladie de Perlizaeus-Merzbacher, la maladie d'Alzheimer, la maladie de Parkinson, la sclérose latérale amyotrophique, la paralysie supranucléaire progressive, la maladie de Pick, la maladie de Huntington, l'ataxie spinocérébelleuse de type I, l'atrophie musculaire spinale et bulbaire, le pallidoluysien dentatorubrale, la dystrophie myotonique, la maladie de Creutzfeldt-Jakob héréditaire (due à un défaut de traitement des protéines prions), la maladie de Fabry, la maladie cholestatique du foie (cirrhose biliaire primaire (CBP)), la cholangite sclérosante primitive (CSP) et le syndrome de Straussler-Scheinker.

2. Composé pour une utilisation selon la revendication 1, le patient ayant une ou plusieurs mutations de CFTR choisies dans le groupe constitué par ΔF508, S549N, G542X, G551D, R117H, N1303K, W1282X, R553X, 621+1G>T, 1717-1G>A, 3849+10kbC>T, 2789+5G>A, 3120+1G>A, 1507del, R1162X, 1898+1G>A, 3659delC, G85E, D1152H, R560T, R347P, 2184insA, A455E, R334W, Q493X, E56K, P67L, R74W, D110E, D110H, R117C, G178R, E193K, L206W, R347H, R352Q, A455E, S549R, G551S, D579G, S945L, S997F, F1052V, K1060T, A1067T, G1069R, R1070Q, R1070W, F1074L, G1244E, S1251N, S1255P, D1270N, G1349D, et 2184delA CFTR.

3. Composé pour une utilisation selon l'une quelconque des revendications 1-2, le patient ayant une mutation ΔF508 une mutation G542X.

4. Composé pour une utilisation selon l'une quelconque des revendications 1 à 3, le patient ayant une mutation ΔF508 homozygote.

5. Composé pour une utilisation selon l'une quelconque des revendications 1 à 4, la maladie étant la fibrose kystique.

6. Composé pour une utilisation selon l'une quelconque des revendications 1 à 5, le patient étant un patient humain.

7. Composé amplificateur de CFTR pour une utilisation dans un procédé de traitement de la fibrose kystique chez un patient homozygote pour la mutation ΔF508 ou ayant une mutation ΔF508/G542X, le procédé comprenant une administration au patient d'une combinaison efficace, séquentiellement ou sensiblement simultanément, du composé amplificateur de CFTR, d'un composé correcteur de CFTR et d'un composé potentiateur de CFTR,
le composé amplificateur étant représenté par : 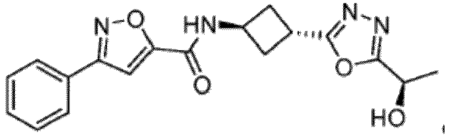 ou un sel pharmaceutiquement acceptable correspondant ;
le composé correcteur étant représenté par : 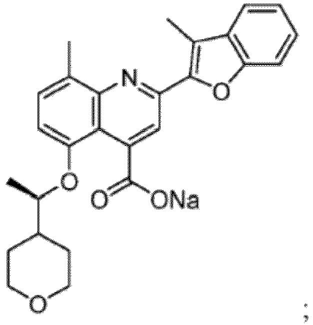 et
le composé potentiateur étant représenté par : 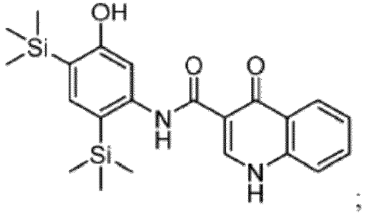
ou un sel pharmaceutiquement acceptable correspondant.

8. Composé pour une utilisation selon l'une quelconque des revendications 1 à 7, le procédé comprenant en outre une administration d'un correcteur de CFTR supplémentaire.

9. Composé pour une utilisation selon la revendication 8, le correcteur de CFTR étant choisi dans le groupe constitué par l'acide (3-(6-(1-(2,2-difluorobenzo[d][1,3]dioxol-5-yl)cyclopropanecarboxamido)-3-méthylpyridin-2-yl)benzoïque (lumacaftor), le lumacaftor deutéré, le ((R)-1-(2,2-difluorobenzo[d][1,3]dioxol-5-yl)-N-(1-(2,3-dihydroxypropyl)-6-fluoro-2-(1-hydroxy-2-méthylpropan-2-yl)-1H-indol-5-yl)cyclopropane-1-carboxamide (tezacaftor), le tezacaftor deutéré, VX-152, VX-440, VX-445, VX-659, GLPG2222, GLPG2851, GLPG2737, GLPG3221 et VX-983.
